(19) Europäisches Patentamt
European Patent Office
Office européen des brevets

(11) EP 3 288 517 B1

(12) **EUROPÄISCHE PATENTSCHRIFT**

(45) Veröffentlichungstag und Bekanntmachung des Hinweises auf die Patenterteilung:
**17.07.2019 Patentblatt 2019/29**

(21) Anmeldenummer: **16720803.2**

(22) Anmeldetag: **29.04.2016**

(51) Int Cl.:
***A61H 35/00*** *(2006.01)* ***A61H 33/14*** *(2006.01)*
***A47K 3/28*** *(2006.01)*

(86) Internationale Anmeldenummer:
**PCT/EP2016/059589**

(87) Internationale Veröffentlichungsnummer:
**WO 2016/174192 (03.11.2016 Gazette 2016/44)**

(54) **MEDIZINISCHE BADEVORRICHTUNG**

MEDICAL BATHING DEVICE

BAIGNOIRE MÉDICALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.04.2015 EP 15165744**

(43) Veröffentlichungstag der Anmeldung:
**07.03.2018 Patentblatt 2018/10**

(73) Patentinhaber: **BSN Medical GmbH**
**20253 Hamburg (DE)**

(72) Erfinder:
- **HEMMRICH, Karsten**
**40667 Meerbusch (DE)**
- **ARSHI, Annahit**
**22527 Hamburg (DE)**
- **SCHULZE, Christian**
**21255 Tostedt (DE)**

(74) Vertreter: **FARAGO Patentanwälte**
**Thierschstraße 11**
**80538 München (DE)**

(56) Entgegenhaltungen:
**EP-A1- 1 903 003**
**WO-A1-2013/040415**
**WO-A1-2013/063354**
**JP-A- H01 223 915**
**US-A- 5 848 998**
**US-A1- 2014 088 490**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 288 517 B1

## Beschreibung

### Gegenstand der Erfindung

Die vorliegende Erfindung betrifft eine medizinische Badevorrichtung zur Herstellung und Duschanwendung einer wirkstoffhaltigen Badelösung und insbesondere einer NO-haltigen Badelösung, die bevorzugt mit einem mehrstufigen Verfahren hergestellt wird. Die Erfindung betrifft zudem eine Badevorrichtung unter Anwendung dieses NO-Herstellungsverfahrens und die Badevorrichtung in ihrer Verwendung zur Behandlung von Erkrankungen, insbesondere von diabetisch bedingten Durchblutungsstörungen und Wunden der unteren Extremitäten.

### Hintergrund der Erfindung

Aus dem Stand der Technik sind zahlreiche Verfahren und Vorrichtungen zur Herstellung von NO bekannt.

Dokument WO 2013/040415 A1 offenbart eine Medizinische Badevorrichtung nach dem Oberbegriff des Anspruchs 1.

Gemäß der EP 1 903 003 A1 kann NO durch Photolyse einer photolabilen NO-Vorstufe hergestellt werden, wobei die die Reaktion unter Anwesenheit von Radikalfängern und Antioxidantien zur Bildung von hochreinem NO verläuft. Bei diesem Verfahren ist in der Anwendung auf die NO-Erzeugung innerhalb von Flüssigkeiten in der Regel nur mit einem langsamen Anfluten der NO-Konzentration zu rechnen.

Gemäß der WO2013/063354 kann ein NO-freisetzendes Fußbad hergestellt werden, indem ein Polysiloxan-Polymer, das mit Diazeniumdiolatgruppen derivatisiert ist, der Badlösung zugegeben wird. Dieses reagiert dann mit Wasser unter Bildung von NO. Da die NO-Generierung hierbei durch einen spontanen Zerfall der Polymerseitengruppen erfolgt, kann die Freisetzungskinetik hierbei nur unzureichend gesteuert werden. Zudem dauert es bei diesem Verfahren eine geraume Zeitspanne, bis ein therapeutisch relevanter NO-Spiegel aufgebaut ist.

Es besteht daher noch Bedarf an neuen Badevorrichtungen zur Herstellung NO-haltiger Lösungen, in denen in kontrollierter Weise NO in hoher Reinheit erzeugt werden kann und die eine sichere Badeanwendung erlauben.

Aufgabe der Erfindung ist es daher, eine Badevorrichtung zur Herstellung und Anwendung einer wirkstoffhaltigen Badelösung bereitzustellen, die bezüglich mindestens einer der oben genannten Nachteile verbessert ist.

### Zusammenfassung der Erfindung

Die vorliegende Erfindung stellt eine Medizinische Badevorrichtung gemäß dem Anspruch 1, eine Verwendung der medizinischen Badevorrichtung gemäß dem Anspruch 15 und ein kosmetisches Verfahren gemäß dem Anspruch 20 bereit.

Spezifische Ausgestaltungen der Erfindung sind Gegenstand weiterer abhängiger und unabhängiger Ansprüche.

Das erfindungsgemäße Verfahren vereinigt mehrere entscheidende Vorteile gegenüber den aus dem Stand der Technik bekannten Verfahren.

Die Verwendung einer Duschvorrichtung hat sich im Rahmen der wirkstoffbasierten Anwendung als besonders vorteilhaft herausgestellt. Im Gegensatz zu einem Tauchbad kann auch mit einer geringen Menge an Badelösung der zu behandelnde Körperteil vollständig benetzt werden.

Entsprechend geht die Badeanwendung mit einem verringerten Verbrauch an Wasser und Wirkstoff einher, und erlaubt so eine kostengünstige Anwendung.

Aufgrund der Duschvorrichtung kann sie flexibel auf die jeweilige Behandlungssituation angepasst werden.

Zudem erlaubt dies eine einfache Regeneration der wirkstoffhaltigen Badelösung, die insbesondere bei Wirkstoffen wichtig ist, die schnell abgebaut werden oder vom Körper aufgenommen werden, wie es beispielsweise für NO gegeben ist. So sinkt bei dem Überfließen der zu behandelnden Körperextremitäten der NO-Gehalt der Badelösung, sei es durch NO-Abbau oder Diffusion in das Hautgewebe und die bodenseitig anfallende NO-arme Badelösung kann in das Reaktionsgefäß zur erneuten NO-Anreicherung geleitet werden und danach wieder der Duschvorrichtung zugeführt werden.

Es gibt zahlreiche Ausgestaltungen für eine Duschvorrichtung (Schwalldusche, Tropfendusche, Strahldusche), die an die jeweilige Anwendung angepasst werden können.

Die erfindungsgemäße Badevorrichtung ist aufgrund der geringen Größe und der Möglichkeit, sie mit Rollen oder Rädern auszugestalten, besonders gut für eine mobile Anwendung geeignet.

### Die Erfindung im Einzelnen

In einer Ausführungsform der Erfindung entspricht bei der medizinischen Badevorrichtung das Gefäß zur Aufnahme der verbrauchten Badelösung dem Reaktionsgefäß. Hierdurch resultiert eine besonders kompakte und platzsparende Ausführung, die auch sehr ökonomisch arbeitet, da die verbrauchte Badelösung nach Regeneration, bzw. Neuherstellung der wirkstoffhaltigen Badelösung wieder der Duschvorrichtung zugeführt werden kann.

In einer weiteren Ausführungsform der Erfindung stellen das Reaktionsgefäß und die Behandlungskammer eigenständige Gefäße dar, die dann bevorzugt über eine Flüssigkeitsleitung miteinander verbunden sind.

Zweckmäßigerweise ist das Reaktionsgefäß als geschlossener Behälter ausgebildet, der jeweils wenigstens einen Zulauf und einen Ablauf für die Badelösung aufweist. Ein solcherart geschlossener Behälter erlaubt eine kontrollierte und reproduzierbare Reaktionsführung zur optimierten Herstellung von wirkstoffhaltigen Badelösungen. Bei Herstellungsverfahren, die mit der Herstellung von toxischen oder unverträglichen Zwischen- oder Endprodukten einhergehen, wird dadurch auch die Freisetzung dieser Produkte verhindert. Weiterhin kann bei der Verwendung von UV-Licht im Rahmen der Wirkstoffherstellung (z.B. durch die Photolyse von NO-Donoren) dadurch das Austreten der UV-Strahlung unterbunden werden.

Gemäß der Erfindung umfasst die Badevorrichtung zusätzlich eine oder mehrere Lichtquellen zur Photolyse von NO-Donoren in der Badelösung. Diese sind an oder in dem Reaktionsgefäß angeordnet.

Gemäß der Erfindung handelt es sich bei diesen Lichtquellen um UV-Lichtquellen.

Gemäß der Erfindung handelt es sich bei der Duschvorrichtung um einen tragbaren Duschkopf.

Zweckmäßigerweise umfasst die Behandlungskammer zusätzlich eine Auflage zum Aufsetzen oder Auflegen der mindestens einen Körperextremität. Diese Auflage enthält bevorzugterweise mindestens eine Öffnung zum Abfließen der Badelösung. Dies hat den Vorteil, dass die auf dieser Auflage aufsetzende oder aufliegende Körperextremität, bevorzugt eine Hand, ein Arm, ein Fuß oder ein Bein nicht direkt in der wirkstoffhaltigen , bevorzugt NO-haltigen, Badelösung eintaucht, sondern seine NO-Zufuhr ausschließlich durch die Duschvorrichtung erhält, so dass hiermit bei gleichmäßiger NO-Zufuhr eine besonders gute Kontrolle der auf den Körper einwirkenden NO-Exposition resultiert. In zweckmäßigerweise ist diese Auflage als Gitter oder Sieb ausgestaltet, so dass die Badelösung schnell und ohne Pfützenbildung abfließen kann. Hierdurch wird verhindert, dass die Körperextremität zu lange mit der wirkstoffhaltigen Badelösung in Kontakt kommt. Eine Ausgestaltung als Sieb oder Gitter hat den Vorteil, dass bei einer entsprechend engen Maschenweite auch Hautpartikel oder Wundbestandteile festgehalten werden und nicht in das System gelangen. Zweckmäßigerweise stellt die Auflage einen Einwegartikel dar, der nach der Badebehandlung mit den aufgefangenen Partikeln entsorgt werden kann.

In einer weiteren Ausführungsform enthält die medizinische Badevorrichtung in der Behandlungskammer unterhalb der Auflage eine oder mehrere Trennabschnitte mit mindestens einer Öffnung zum Abfließen der Badelösung, wobei die mindestens eine Öffnung kleiner ist als die die mindestens eine Öffnung der Auflage. Hierdurch können Partikel, die durch die Öffnungen der Auflage hindurchgelassen worden sind, in den darunter befindlichen Trennabschnitten aufgefangen werden und so die Badelösung vor dem Eintritt in das Reaktionsgefäß vorgereinigt werden. Bei mehreren Trennabschnitten weisen diese in bevorzugter Weise von oben nach unter eine abnehmende Größe der Öffnungen auf, so dass hierdurch eine stufenweise Filtration der in der Badelösung enthaltenden Partikel resultiert.

In einer weiteren Ausführungsform ist das Gefäß zur Aufnahme der verbrauchten Badelösung neben, unterhalb oder oberhalb der Behandlungskammer angebracht und mit der Behandlungskammer flüssigkeitsführend verbunden. In bevorzugter Weise ist hierbei die Behandlungskammer bevorzugt von dem Gefäß abnehmbar, wie es beispielsweise durch eine Steck-, Bajonett- oder Schraubverbindung realisierbar ist.

In einer bevorzugten Ausführungsform ist das Gefäß zur Aufnahme der verbrauchten Badelösung unterhalb der Behandlungskammer angebracht und mit der Behandlungskammer flüssigkeitsführend verbunden. In bevorzugter Weise ist hierbei die Behandlungskammer bevorzugt von dem unteren Gefäß abnehmbar, wie es beispielsweise durch eine Steck-, Bajonett- oder Schraubverbindung realisierbar ist.

In einer weiteren Ausführungsform weist die medizinische Badevorrichtung bodenseitig Rollen oder Räder auf, so dass sie leicht auf dem Untergrund bewegbar ist.

In einem weiteren Aspekt stellt die Erfindung eine medizinische Badevorrichtung zur Behandlung von Körperextremitäten mit einer wirkstoffhaltigen Badelösung bereit, die Folgendes umfasst:

(a) Einen an einen Wasseranschluss direkt oder über eine Leitung anschließbares Reaktionsgefäß zur Erzeugung einer wirkstoffhaltigen Badelösung; und
(b) einen tragbaren Duschkopf zur Abgabe der wirkstoffhaltigen Badelösung;

wobei der Duschkopf über eine Leitung mit dem Reaktionsgefäß verbunden ist oder das Reaktionsgefäß umfasst.

In dieser Ausführungsform kann auf eine Pumpe verzichtet werden, da der Wasseranschluss selber für einen Druckbeaufschlagung der Flüssigkeit sorgt.

Das Reaktionsgefäß kann bei dieser Ausführungsform in verschiedener Weise in das System eingebunden werden:

1. Das Reaktionsgefäß kann direkt an dem Wasseranschluss angebracht werden;
2. Das Reaktionsgefäß kann über eine Leitung, bevorzugt über einen Schlauch mit dem Wasseranschluss verbunden sein; oder
3. Das Reaktionsgefäß kann in dem Duschkopf enthalten sein.

Bei der Ausführungsform gemäß des zweiten Aspekts der Erfindung kann der Körperteil in einer normalen (Dusch)wanne behandelt werden, so dass die Badelösung über den hierein enthaltenen Ausguss entsorgt wird. Alternativ kann die Duschbehandlung in einer erfindungsgemäßen Behandlungskammer erfolgen, wie sie vorab genannt ist und hierbei auch die Ausgestaltungen wie Auflage oder Trennabschnitte enthalten und darüber hinaus mit einem Gefäß zur Aufnahme der verbrauchten Badelösung verbunden sein.

In einer bevorzugten Ausführungsform umfasst diese medizinische Badevorrichtung auch einen, bevorzugt dem Reaktionsgefäß vorgelagerten, Druckregulierer, der den initialen Druck des Wasseranschlusses auf den gewünschten Enddruck reduziert.

In einer weiteren Ausführungsform ist bei dieser medizinischen Badevorrichtung dem Reaktionsgefäß auch ein Filter vorgeschaltet, um das Wasser, das typischerweise Leitungswasser darstellt, vor der Herstellung der Badelösung zu filtern.

In einer bevorzugten Ausführungsform der Erfindung ist die wirkstoffhaltige Badelösung der erfindungsgemäßen medizinischen Badevorrichtungen eine Stickstoffmonoxid (NO)-haltige Badelösung.

<u>Duschvorrichtung</u>

Erfindungsgemäß umfasst die Badevorrichtung zur Behandlung des Körpers oder Körperteils eine Duschvorrichtung.

Eine solche Duschvorrichtung verringert das Risiko der (Re-)kontamination von Wunden durch Mikroben aus der Badelösung oder benachbarten Hautarealen, da die kontaminierte Badelösung direkt von der Hautstelle abfließt und durch neue, nichtkontaminierte Badelösung ersetzt wird.

Im Gegensatz zu einem Eintauchbad wird die Haut nicht übermäßig aufgeweicht. Da zudem keine Eintauchbehälter gefüllt werden muss, ist diese Art der Anwendung auch schneller und die Behandlung kann direkt nach Generierung der wirkstoffhaltigen Badelösung beginnen.

Bei instabilen Wirkstoffen, wie beispielsweise NO, erlaubt die Duschanwendung in einfacherer Weise die Herstellung von Badelösungen mit konstanter NO-Konzentration. Eine Duschvorrichtung erlaubt eine flexiblere Anwendung, wobei die Behandlung auf die notwendigen Körperbereiche fokussiert werden kann.

In einer Ausführungsform der Erfindung umfasst die Duschvorrichtung mehrere voneinander beabstandete Duschköpfe, die bevorzugt über eine gemeinsame Flüssigkeitsleitung miteinander verbunden sind.

In einer weiteren Ausgestaltung ist an dem Duschkopf eine Umstellvorrichtung vorgesehen, durch die mit dem Duschkopf verschiedene Strahlarten erzeugt werden können, beispielsweise ein normaler Wasserstrahl und ein Brausestrahl.

In einer besonderen Ausführungsform wird durch die Duschvorrichtung ein pulsierender Wasserstrahl erzeugt, der als Massagestrahl die therapeutische Wirkung gefäßerweiternder Wirkstoffe, wie beispielsweise NO, zusätzlich vorteilhaft unterstützt.

In einer besonderen Ausführungsform nutzt der Duschkopf das Prinzip der Venturidüse und ermöglicht die Vermischung einer wirkstofffreien Badelösung mit der wirkstoffhaltigen Badelösung. In bevorzugter Wiese ist hierbei der in dem Duschkopf integrierten Behälter für die wirkstoffhaltige Badelösung mit der Wasserzufuhr verbunden. Durch die Verbindung mit der Venturidüse, die sich am Behälter befindet, wird die wirkstoffhaltige Badelösung aus dem Behälter transportiert und in dieser Mischung an die Duschkopflöcher weitergeleitet. Die Vermischung erfolgt beispielsweise durch Betätigung eines Schalters, der die Verbindung zwischen der Venturidüse und dem Wirkstoff-Badelösungsbehälter öffnet.

Die Duschvorrichtung ist zweckmäßigerweise so ausgestaltet, dass die Freisetzung des Wirkstoffs, insbesondere bei gasförmigen Wirkstoffen wie NO, aus der Badelösung in die Luft verhindert wird. Hierzu kann beispielsweise der Duschkopf eine randseitige Luftansaugung aufweisen, so dass das aus dem Wasserstrahlen austretende NO sofort abgesaugt wird und entweder im Duschkopf der Badelösung wieder zugeführt wird oder aus dem System (z. durch Filtration, Adsorption oder Abbau) entfernt wird.

Dies kann auch durch einen Duschkopf mit zwei unterschiedlichen Austrittsbereichen gewährleistet werden, wobei ein erster, innerer Bereich des Duschkopfs für die wirkstoffhaltige Badelösung vorgesehen ist und ein zweiter ringförmiger Austrittsbereich, der den inneren Bereich umschließt, für eine wirkstofffreie Badelösung vorgesehen ist. Dieser zweite Bereich bildet einen "Mantel" aus wirkstoff-freier Badelösung und sorgt dafür, dass der aus der Badelösung des ersten Bereichs austretende Wirkstoff hierein gelöst wird und nicht in die Umgebung gerät.

In einer weiteren Ausgestaltung der Erfindung ist die Duschvorrichtung nicht als Duschkopf gestaltet, sondern

als Schlauch oder Rohr mit Austrittsöffnungen, wobei der Schlauch oder das Rohr bevorzugt als Ring oder als Spirale ausgeformt sind. In einer bevorzugten Ausführungsform ist der Ring oder die Spirale an der Innenwand einer Duschkammer der Duschvorrichtung angebracht und die Austrittslöcher weisen hierbei nach innen.

In einer bevorzugten Ausgestaltung, ist die Duschvorrichtung so ausgestaltet, dass sie auf dem zu behandelnden Körperteil aufgelegt oder befestigt werden kann und so in bevorzugter Weise einen Wasserfilm aufbaut, der an dem Körperteil abläuft. Diese Ausgestaltung hat den Vorteil, dass sie mit besonders geringen Mengen an wirkstoffhaltiger Badelösung auskommt und durch die Filmbildung (im Gegensatz zu einer Sprühvorrichtung) die Freisetzung von potenziell toxischen Wirkstoffen in die Umwelt besonders gut verhindert. Für diese Ausgestaltung kann der Schlauch oder der (Halb)ring partiell oder vollständig um das zu behandelnde Körperteil herumgeführt werden und beispielsweise durch eine leichte Klemmwirkung an diesem arretiert werden. In einer alternativen Ausgestaltung, kann die Duschvorrichtung auch als Bügel ausgestaltet sein, der in seiner Form an das zu behandelnde Körperteil angepasst ist.

In einer besonderen Ausführungsform ist an dem oben erwähnten Ring, Schlauch- oder Bügel ein Duschvorhang befestigt. Bei der körperseitigen Befestigung dieser Duschvorrichtungen verhindert dieser körpernahe Duschvorhang in zusätzlichem Maße die Freisetzung des Wirkstoffs.

In einer weiteren Ausgestaltung ist die Duschvorrichtung als Drainagestrumpf, Bandage oder Handschuh ausgestaltet und erlaubt so eine gezielte körperseitige Freisetzung des Wirkstoffs.

Des Weiteren kann die Duschvorrichtung mit einer oder mehreren Körperabdeckungen kombiniert werden, so dass nur der zu behandelnde Bereich für die Duschanwendung zugänglich ist. In einer bevorzugten Ausführungsform kann diese Abdeckung eine oder mehrere Aussparungen für den zu behandelnden Körperbereich aufweisen.

In einer weiteren Ausgestaltung ist bei der Duschvorrichtung die Austrittsöffnung schlitzförmig ausgestaltet, so dass die Duschvorrichtung als Schwalldusche fungiert. Gegenüber einem Duschkopf mit vielen einzelnen Wasserstrahlen, führt so eine Schwalldusche zu einer geringeren Freisetzung des Wirkstoffs in die Umgebung.

Zweckmäßigerweise ist die Duschvorrichtung mit einem Schalter ausgestattet, der die Wasserzufuhr regelt.

Darüber hinaus kann die Duschvorrichtung noch einen Druckregler aufweisen, der den Wasserdruck und damit die austretende Wassermenge reguliert.

In bevorzugter Weise ist die wirkstoffhaltige Badelösung bei den vorab genannten Ausgestaltungen der Duschvorrichtung eine NO-haltige Badelösung.

In einer Ausführungsform der Erfindung wird durch die Badevorrichtung ein sprudelndes Bad bereitgestellt. Dies kann durch Einblasen eines Gases erzeugt werden oder durch eine chemische Reaktion, bei der ein Gasbildner wie beispielsweise ein Carbonatsalz durch Ansäuerung der Badelösung zur Freisetzung von $CO_2$-Gas induziert wird.

In einer weiteren Ausführungsform der Erfindung ist die Badevorrichtung mit einer Vorrichtung versehen, die die Freisetzung von NO in die Umwelt verringert oder gänzlich verhindert. Dies kann eine mechanische Abtrennung sein, die beispielsweise in Form einer Haube oder eine Abdeckfolie das Reaktionsgefäß und/oder die Behandlungskammer abdeckt, wobei sie eine Aussparung für den einzutauchenden Körperteil vorsieht. Alternativ kann es sich um eine Absaugvorrichtung handeln, die das aus der Badelösung freigesetzte NO absaugt und entweder dem Badelösung zuführt oder das NO abbaut bzw. abfiltriert.

In einer bevorzugten Ausführungsform ist das Reaktionsgefäß ein im Wesentlichen geschlossenes, d.h. hermetisch von der Umwelt abgeschottetes System, das lediglich mit dem Behälter zur Aufnahme des zu behandelnden Körperteils in Verbindung steht. Dadurch wird gewährleistet, dass das in dem Reaktionsgefäß erzeugte NO bevorzugt an die Badelösung abgegeben wird und nicht in die Umwelt gelangt.

In einer weiteren Ausführungsform umfasst die Badevorrichtung einen NO-Sensor, so dass als Rückkopplung auf den gemessenen NO-Wert das Ausmaß der NO-Generierung flexibel angepasst werden kann.

Dieser NO-Sensor als Messvorrichtung zur Quantifizierung des NO kann in dem Reaktionsgefäß, in dem Behandlungsbehälter oder sogar auf der Außenseite der Badevorrichtung angebracht sein. In einer besonderen Ausführungsform sorgt die NO-Sensor-assoziierte Steuerung dafür, dass bei Überschreiten eines kritischen NO-Wertes die Badevorrichtung die NO-Generierung gänzlich einstellt.

In einer Ausführungsform der Erfindung wird das Reaktionsgefäß so angesteuert, dass in der Badelösung der Gehalt an NO über den Zeitraum der Behandlung konstant gehalten wird.

In einer alternativen Ausführungsform der Erfindung wird das Reaktionsgefäß so angesteuert, dass der Gehalt an NO über den Zeitraum der Behandlung ansteigt oder abfällt.

In einer weiteren Ausführungsform der Erfindung wird die Badevorrichtung zur Badebehandlung von Gegenständen, Vorrichtungen oder Instrumenten verwendet. Durch die Einwirkung von NO auf diese Gegenstände können diese gereinigt oder desinfiziert werden, die mikrobielle Belastung reduziert werden oder ein Biofilm verringert oder sogar entfernt werden.

In einer bevorzugten Ausführungsform wird die Badevorrichtung hierbei zur Reinigung oder Desinfektion von medizinischen oder chirurgischen Instrumenten verwendet.

In einer Ausführungsform der Erfindung ist die Badevorrichtung so ausgestaltet, dass in diese ein Nachfüllbehälter eingesetzt werden kann, der dann beispielsweise die fertige oder halbfertige Badelösung enthält. Hierbei kann

die fertig formulierte Badelösung durch den Behälter passend in die Eintauchvorrichtung eingesetzt werden und durch die herstellungsseitig vorgegebene Formulierung wird gewährleistet, dass die therapeutisch optimale Formulierung vorliegt.

In einer weiteren Ausführungsform werden die Inhaltstoffe der Badelösung in bevorzugt vorportionierter Form (sog. Verpackungseinheit) der wässrigen Flüssigkeit hinzugegeben. Da die erfindungsgemäße NO-Generierung auch mit herkömmlichem Leitungswasser möglich ist, kann so der Anwender auf dieses Leitungswasser zurückgreifen und mit den Inhaltsstoffen, die beispielsweise Puffersubstanz, Salze, NOD und Antioxidans umfassen, mischen und so zu einer gebrauchsfertigen Badelösung gelangen.

Bei der vorportionierten Form liegen die Inhaltsstoffe bevorzugt in fester Form vor. So können sie als Puder, Pulver, Granulat, Tablette, Filmtablette, Dragee, Weichgelatinekapsel, Hartgelatinekapsel, Oblong, Caplet, Brausetablette oder Pille vorliegen, wobei die Verpackungseinheit zweckmäßigerweise die für jeweils eine Behandlung ausreichenden Menge enthält.

In einer bevorzugten Ausführungsform liegen die Inhaltsstoffe als Brausetablette vor. In dieser Form werden sie schnell gelöst und reichern das Medium zudem mit dem entsprechenden -bevorzugt inerten- Gas (bspw. $CO_2$) an. Diese Darreichungsform ist zudem im Bereich der Badeanwendungen den Anwendern wohlbekannt und besitzt daher auch eine hohe Compliance.

Alternativ können die Inhaltsstoffe in flüssiger oder halbfester Form vorliegen. Halbfeste Formen umfassen beispielsweise: Suspension, Emulsion, Paste, Creme, Salbe, Gel oder Lotion. Die Vorportionierung als Verpackungseinheit kann beispielsweise durch die Verpackung in Ampullen, Flaschen, Säckchen oder Tuben gewährleistet werden.

In einer weiterhin bevorzugten Ausführungsform ist die Verpackungseinheit so ausgestaltet, dass sie von der Form her eine fehlerfreie Anwendung in der Badevorrichtung ermöglicht. So ist die Form in bevorzugter Weise als Kartusche ausgestaltet, die nur in einer Orientierung in die Badevorrichtung befestigbar ist. Darüber hinaus kann diese Kartusche mit einem Arretierungsmechanismus ausgestaltet sein, der nur nach korrekter Arretierung in der Badevorrichtung die Inhaltsstoffe in der gewünschten Weise freigibt. Zweckmäßigerweise kann die Badevorrichtung hierbei mit einem Sensor ausgestattet sein, der eine inkorrekte Orientierung bzw. Arretierung der Kartusche detektiert und dem Anwender anzeigt.

In einem weiterem Aspekt stellt die Erfindung einen Kit bereit, der eine Verpackungseinheit für eine Behandlung umfasst, wobei diese Verpackungseinheit eine pulverförmige, gelförmige oder flüssige Zusammensetzung enthaltend NOD, Puffersubstanz, Antioxidans und optional ein Lösungsmittel aufweist.

Zur Steuerung der Behandlungsdauer kann die Badevorrichtung in bevorzugter Weise eine Zeitsteuerungseinheit umfassen, die nach einer fest vorgegebenen oder bevorzugt flexibel programmierbaren Zeit die NO-Generierung abschaltet.

Darüber hinaus kann die Badelösung einen Farbstoff enthalten, der nach einer bestimmten Zeit eine Farbveränderung erfährt, so dass der Benutzer über das Ende des Behandlungszeitraums informiert ist.

Weiterhin kann die Badevorrichtung auch eine Vorrichtung zur Messung der Durchblutung umfassen, der anhand des Therapieerfolges eine besonders gute Steuerung der Behandlungsdauer und/oder Behandlungsintensität erlaubt. Dem Fachmann sind zahlreiche Vorrichtungen zur Messung der Durchblutung bekannt. Beispiele hierfür sind Gefäßtachometer, oder der in der WO 97/46853 offenbarte Mikrosensor. Dieser Sensor umfasst einen Indikator-durchlässigen Einsatz, der in einer Öffnung eines Indikator-Behälters, der durch ein Behältnis gebildet ist, angeordnet ist, wodurch der Einsatz einen durchlässigen Wand-Abschnitt des Behälters bildet.

Als Surrogatparameter für die Hautdurchblutung können weitere vaskulär bedingte Messparameter wie die Rötung der Haut oder die Hauttemperatur dienen, für die entsprechende Messmethoden und -geräte aus dem Stand der Technik bekannt sind.

In einer bevorzugten Ausführungsform ist die Badevorrichtung mit einer UV-Strahlungsquelle versehen, deren UV-Strahlung durch photolytischen Zerfall das NO direkt in der Badelösung generiert. Dies hat den Vorteil, dass die Badelösung in einem abgeschlossenen Kompartiment vorliegen kann und zudem die NO-Generierung in kontrollierter und reproduzierbarer Weise ablaufen kann.

Bevorzugterweise wird zur NO-Generierung die Badelösung in der Badevorrichtung in einem flachen Reaktionsgefäß von der Strahlungsquelle angestrahlt.

So ist für die photolytische Spaltung ein Reaktionsgefäß mit einer Schichtdicke von zwischen 1 und 20 mm, bevorzugt von zwischen 2.5 und 10 mm und besonders bevorzugt von zwischen 5 und 7.8 mm geeignet. Es zeigte sich, dass eine entsprechend dimensionierten Schichtdicke durch optimale Ausnutzung der UV-Strahlung zu einer hohen Ausbeute an NO führt.

Zweckmäßigerweise ist das Material des Reaktionsgefäßes für UV-Strahlung durchlässig. Aufgrund seiner Kenntnis der UV-Durchlässigkeit wird der Fachmann die geeigneten Materialien für das Reaktionsgefäß auswählen. Bei UV-Strahlung im $UV_A$-Bereich (315 bis 380 nm) kann noch herkömmliches Natron-Kalk-Glas verwendet werden, bei höherenergetischer Strahlung bis 290 nm kann Borosilikatglas zum Einsatz kommen, und bei UV-Strahlung unterhalb von 290 nm ist Quarzglas geeignet.

Auch UV-durchlässige Kunststoffe wie Polymethylpenten (PMP), modifiziertes Polymethylmethacrylat (PMMA), modifiziertes Polyvinylbutyral (Trosivol UV+®) können als Material für das Reaktionsgefäß verwendet werden.

In einer bevorzugten Weise ist das Reaktionsgefäß so ausgeformt, dass es mit seiner der Strahlungsquelle zugewandten Fläche einen definierten, gleichbleibenden Abstand aufweist. Bei einer röhrenförmigen Strahlungsquelle ist das Reaktionsgefäß entsprechend als Hohlzylinder ausgeformt, in dessen Zentrum die Röhre positioniert ist. Die Badelösung wird hierbei zweckmäßigerweise an einem Ende des Zylinders zugeführt, strömt über die Länge des Zylinders an der UV-Strahlungsquelle vorbei, wobei es sich zunehmend mit NO anreichert und wird am anderen Ende des Zylinders entnommen, um dem Behandlungsbehälter zugeführt zu werden.

Alternativ kann das Reaktionsgefäß auch ein Rohr sein, das als Spirale mit definiertem Innendurchmesser ausgeformt ist, wobei die röhrenförmige UV-Quelle im Zentrum der Spirale angeordnet ist. Diese Anordnung ermöglicht einen graduellen Anstieg der NO-Konzentration, wobei die NO-Ausbeute hier bei gleichbleibender Strahlungsintensität durch die Fließgeschwindigkeit in der Spirale gesteuert werden kann.

In einer alternativen Ausgestaltung ist bei einer flächenförmigen Strahlungsquelle (z.B. durch ein LED-Panel) das Reaktionsgefäß als flacher Kasten ausgeformt. Dieser weist bevorzugt diametral angebrachten Zu- und Abflüsse für die Badelösung auf und kann im Inneren auch Trennwände enthalten, die den Fluss der Badelösung in geeigneter Weise steuern können.

In einer weiteren Ausführungsform ist das Reaktionsgefäß auf der von der Strahlungsquelle abgewandten Weise mit einer UV-reflektierenden Beschichtung versehen. Damit kann die Strahlungsausbeute zusätzlich erhöht werden, indem das reflektierte UV-Licht erneut die Badelösung durchqueren kann und hierbei NO photolytisch generieren kann. Dem Fachmann sind entsprechende UV-reflektierende Schichten wie bspw. Aluminium oder dielektrische Schichten bekannt. In einer alternativen Ausführungsform ist die UV-reflektierende Beschichtung nicht auf dem Reaktionsgefäß selbst, sondern getrennt dazu angebracht, z.B. auf der Innenwand der Badevorrichtung.

Erfindungsgemäß umfasst die Badevorrichtung auch ein System zum Umwälzen und/oder Pumpen der Badelösung.

Diese Pumpvorrichtung kann bei der erfindungsgemäßen Badevorrichtung in verschiedener Weise zum Einsatz kommen. So kann sie dazu dienen, die im Reaktionsgefäß hergestellte wirkstoffhaltige Badelösung zu der Duschvorrichtung zu transportieren. Weiterhin kann die Pumpvorrichtung auch dem Reaktionsgefäß vorgeschaltet sein und für den Transport der extern bereitgestellten Flüssigkeit in das Reaktionsgefäß dienen.

Zudem kann auch die flüssigkeitsführende Verbindung zwischen der Behandlungskammer und dem Gefäß oder der Vorrichtung zu Aufnahme der verbrauchten Badelösung eine Pumpvorrichtung umfassen. Hierdurch wird die verbrauchte Badeflüssigkeit aus der Behandlungskammer abgepumpt werden, um sie beispielsweise der Entsorgung oder der Filterung zuzuführen.

In einer alternativen Ausführungsform der Erfindung wird fließt die Badelösung mittels Gravitation von der Behandlungskammer in das Gefäß oder die Vorrichtung zur Aufnahme der verbrauchten Badelösung ab. Das Gefäß oder die Vorrichtung zur Aufnahme der verbrauchten Badelösung ist hierbei unterhalb der Behandlungskammer angeordnet.

Dem Fachmann sind Pump- bzw. Umwälzvorrichtungen aus dem Stand der Technik bekannt und er kann anhand der relevanten Parameter wie Viskosität der Badelösung, erforderliche Pumpleistung, Volumen des Reaktionsgefäßes und der Behandlungskammer, Sprüh/Duschleistung des Sprüh- oder Duschkopfs die passende Vorrichtung auswählen.

Als Pumpvorrichtungen kommen hier beispielsweise in Betracht: Schlauchpumpen, Membranpumpen, Kolbenpumpen, magnetgekoppelte Pumpen und Impellerpumpen.

In einer Ausführungsform Erfindung umfasst bei der medizinischen Badevorrichtung die flüssigkeitsführende Verbindung zwischen der Behandlungskammer und dem Gefäß oder der Vorrichtung zu Aufnahme der verbrauchten Badelösung eine Filtervorrichtung und/oder eine Absorptionsvorrichtung zur Aufreinigung der verbrauchten Badelösung. Diese Filtervorrichtung kann je nach Reinigungszweck unterschiedlich gestaltet sein. Durch einen Partikelfilter können so bspw. ungelöste Partikel der Badelösung, Schwebstoffe, Haut und Wundpartikel abgefangen werden.

Durch einen Sterilfilter können (unter Umständen pathogene) Mikroorganismen, die gerade im Wundbereich anzutreffen sind, und die durch die Duschbehandlung aus der Wunde gespült werden entfernt werden.

Durch einen NO und oder $NO_2$-Filter oder eine NO bzw. $NO_2$-Absorptionsvorrichtung können hier diese Gase aus der Flüssigkeit entzogen werden. Hierzu kann Aktivkohle, Zeolithe oder Polyphenylensulfid Polymere (wie bspw. "noXon" der Firma Hoechst AG, Frankfurt, BRD) verwendet werden.

In bevorzugter Weise ist der Filter so ausgestaltet, dass er den NO-Donor, der in bevorzugter Weise ein Nitritsalz ist, aus der Badelösung entfernt. Eine derart gefilterte Badelösung kann dann ohne Probleme als Haushaltsabwasser, also bspw. über den Ausguss entsorgt werden.

In bevorzugter Weise ist der Filter so ausgestaltet, dass er neben den dem NO-Donor auch die schädlichen Stickstoffoxidspezies, die hier vor allem NO und NO2 darstellen, aus der Badelösung entfernt.

In einer weiteren Ausführungsform der Erfindung umfasst das Gefäß oder die Vorrichtung zur Aufnahme der

verbrauchten Badelösung superabsorbierendes Material. Hierdurch kann gerade bei einer geringen Menge an Badelösung die verbrauchte Badelösung gänzlich gebunden werden und damit in einfacher Weise entsorgt werden. Die superabsorbierenden Materialien können in einem Gefäß enthalten sein. Alternativ können sie auch in eine Vorrichtung wie beispielsweise einem textilen Gebilde vorliegen, dann bevorzugt mit flüssigkeitsundurchlässiger Außenhülle, in das dann die verbrauchte Badelösung eingeleitet wird.

In einer weiterhin bevorzugten Ausführungsform enthält der Superabsorber Substanzen, die schädliche oder unerwünschte Bestandteile der Badelösung wie Stickoxide, NO-Donoren und hier insbesondere Nitrit, oder auch bakteriellen Kontaminationen, binden, abbauen oder inaktivieren.

In einer Ausführungsform der Erfindung stellt die Vorrichtung zur Aufnahme der verbrauchten Badelösung eine Flüssigkeitsleitung zur Weiterleitung an eine von der Badevorrichtung getrennte Entsorgungseinheit dar. Damit enthält die erfindungsgemäße Badelösung kein Gefäß bzw. Vorrichtung zum Sammeln der Badelösung, sondern die Flüssigkeitsleitung, die dann bevorzugt mit einer Filtervorrichtung und/oder einer Absorptionsvorrichtung ausgestattet ist, dient zum Abführen der verbrauchten Badelösung aus der medizinischen Badevorrichtung. Die Badelösung kann hierbei in einen externen Tank, z.B. ein Sammelgefäß geleitet werden oder direkt der Entsorgung (Abfluss, Ausguß) zugeführt werden.

Die Badevorrichtung ist in geeigneter Weise mit einer Temperiervorrichtung versehen. Diese erlaubt durch Heizen und/oder Abkühlen eine Einstellung einer ausgewählten Temperatur. Die Temperatur ist einer der Parameter, die die NO-Ausbeute und die Löslichkeit des generierten NO bestimmen. Zudem kann so bei der Badeanwendung eine für die therapeutische Anwendung optimale Badetemperatur eingestellt werden. Dies kann eine für den Anwender angenehme Temperatur zwischen 23°C und 28°C sein, oder eine Temperatur zwischen 10°C und 20°C, die dadurch die Durchblutung der Haut steigert.

Dem Fachmann sind Temperiervorrichtungen aus dem Stand der Technik bekannt und er kann anhand der relevanten Parameter wie Volumen der Flüssigkeit und Aufheiz- und Abkühlgeschwindigkeiten die passende Vorrichtung auswählen.

In einer bevorzugten Ausführungsform ist eine Temperiervorrichtung insbesondere in Kombination mit einer (UV)-Strahlungsquelle erforderlich, da diese zu einem Aufheizen der Badelösung führt. Um einer Überhitzung des Mediums entgegenzuwirken, muss hier die Kühlung bei verlängerter bzw. intensiver Bestrahlung aktiv werden.

In einer weiteren Ausführungsform fungiert die elektromagnetische Strahlungsquelle nicht nur im Rahmen der NO-Generierung, sondern auch als Heizquelle einer Temperiervorrichtung.

Mehrstufiges NO-Herstellungsverfahren

Die NO-haltige Lösung der Badevorrichtung wird in bevorzugter Weise in dem Reaktionsgefäß mit einem Verfahren zur Herstellung von Stickstoffmonoxid (NO) hergestellt, das die folgenden Schritte umfasst:

(a) Bereitstellen eines Trägermediums umfassend mindestens einen pH-labilen NO-Donor;
(b) Einstellen des pH-Werts des Trägermediums auf einen pH-Wert, der die Zersetzung des mindestens einen pH-labilen NO-Donors unter Bildung von NO induziert;
(c) Aufrechterhalten eines die NO-Bildung induzierenden pH-Werts für eine Zeitdauer, die die Bildung einer physiologisch relevanten Menge an NO erlaubt;
(d) Erhöhen des pH-Werts des Trägermediums;
(e) Optionale Zugabe eines weiteren mindestens einen Antioxidans in Schritt (d) oder in einem nachfolgenden Schritt (e);

wobei das Trägermedium in Schritt (a) zusätzlich mindestens ein Antioxidans enthält oder das mindestens eine Antioxidans in Schritt (b) hinzugegeben wird.

Es zeigte sich in überraschender Weise, dass dieses Verfahren den komplementären Anforderungen eine NO-Freisetzungskinetik gerecht wird. So kann im aziden Milieu sehr schnell eine therapeutisch relevante Konzentration an NO in dem Trägermedium aufgebaut werden, die dann nach pH-Werterhöhung über einen längeren Zeitraum in kontrollierter Weise aufrechterhalten werden kann.

Üblicherweise erschwert die kurze Halbwertszeit des NO den therapeutischen Einsatz. Mit dem bei der Vorrichtung bevorzugt verwendeten Verfahren kann trotz der kurzen Halbwertszeit durch eine Stabilisierung des NOs in dem neutralen oder basischen Trägermedium der NO-Spiegel für eine ausreichende Zeitspanne aufrechterhalten werden.

Durch die Anwesenheit von Antioxidantien erlaubt das Verfahren die Herstellung von NO in einer Reinheit, wie sie für die therapeutische oder kosmetische Anwendung erforderlich ist.

Aus dem Stand der Technik sind zahlreiche pH-labile und photolabile NO-Donoren bekannt, wie beispielsweise Nitritsalze, NONOate oder Nitrosothiole, auf die der Fachmann hier zurückgreifen kann.

Aufgrund der hochkontrollierten Steuerung der Freisetzung kann das Verfahren in Badevorrichtungen eingesetzt werden, die das NO nur in sehr geringen Mengen freisetzen. Dies ist insbesondere bei NO als hochpotentem bioaktivem Molekül ein entscheidender Vorteil. Zudem erlaubt dies die Entwicklung eines Fußbads als Medizinprodukt (z.B. als sog. Medical device class III), insofern hier eine Badevorrichtung vorliegt, bei der die Wirkung primär durch die mechanischen oder physikalischen Eigenschaften der Vorrichtung bedingt ist.

Durch einfache Anpassung des Verfahrens bezüglich NO-Donoren, Säuren und Bestrahlungsquellen kann es gezielt an die Behandlungserfordernisse angepasst werden.

Durch das hier vorgestellte Verfahren kann auch auf eine externe Zuführung von NO verzichtet werden.

Bei dem Verfahren handelt es sich um ein einfaches Verfahren mit größtenteils bekannten Substanzen, so dass es nicht nur kostengünstig sowie auf wenig komplexe Art und Weise durchzuführen ist, sondern auch bei geringer Fehleranfälligkeit einfach in der therapeutischen Anwendung ist.

Die mit dem Herstellungsverfahren betriebenen Badevorrichtungen eröffnen im Hinblick auf die kennzeichnenden Parameter und auf die Materialauswahl weitere Freiheitsräume.

Die erfindungsgemäße Badevorrichtung macht sich somit ein zweistufiges Verfahren zu Nutze, bei dem zunächst eine NO-Generierung im sauren Milieu induziert wird und nach einer ausgewählten Zeitdauer der pH-Wert anschließend erhöht wird, um die pHabhängige NO-Neusynthese zu stoppen oder zu verringern und ein NO-haltige Badelösung bereitzustellen.

Durch die pH-Werterhöhung in den bevorzugterweise neutralen oder basischen Bereich unterbleibt die Neugenerierung von toxischen $NO_2$-Radikalen. Durch die erfindungsgemäße Anwesenheit des mindestens einen Antioxidans werden $NO_2$-Radikale und andere bei der NO-Generierung entstehenden Radikale eliminiert, so dass das Trägermedium mit hochreinem NO angereichert ist.

Ausgangspunkt dieses Verfahrens ist ein Trägermedium, das mindestens einen pH-labilen NO-Donor umfasst.

Weiterhin muss das Trägermedium zu dem Zeitpunkt, an dem ein azider pH-Wert die NO-Generierung erlaubt, zusätzlich mindestens ein Antioxidans umfassen. Hierzu kann das Antioxidans bereits im Schritt (a) im Trägermedium enthalten sein. Dies hat den Vorteil, dass die in dem Trägermedium enthaltenen Bestandteile auch schon während der Produktion und/oder Lagerung durch das anwesende, mindestens eine Antioxidans vor unerwünschter Oxidation geschützt sind. Dies kann gerade bei verfahrensgemäßen Vorrichtungen wie Wundauflagen oder Pflastern von Vorteil sein, da hier die Zugabe von weiteren Substanzen schwer möglich ist und diese eine ausreichende Lagerstabilität aufweisen müssen.

Alternativ kann das mindestens eine Antioxidans im Schritt (b) hinzugegeben werden. Dies ist vor allen dann sinnvoll, wenn es in nachteiliger Weise mit der Badelösung oder einem darin enthaltenen Bestandteil interagiert oder selber darin instabil ist. Weiterhin erlaubt dies die Möglichkeit, ein Antioxidans einzusetzen, das gleichzeitig als Säure die NO-Generierung induziert. Beispiele hierfür sind die Ascorbinsäure oder die Harnsäure.

Badelösunq

Als Badelösung kann hier jede Flüssigkeit verwendet werden, die in der Lage ist NO aufzunehmen und auch wieder abzugeben. In bevorzugter Weise ist die Badelösung eine wässrige Flüssigkeit.

NO-Donor

PH-labile NO-Vorstufen (NO-Donoren, NOD) sind im Stand der Technik bekannt und dem Fachmann geläufig.

In einer bevorzugten Ausführungsform der Erfindung sind die pH-labilen NO-Donoren ausgewählt aus der Gruppe enthaltend organische Nitrate, anorganische Nitrate, anorganische Nitrite, organische Nitritester wie Alkylnitrite, Schwefel-, Stickstoff- oder Sauerstoff-Nitrosoverbindungen, NO-Metall-Verbindungen und NO-chelatierende Substanzen.

Beispiele für pH-labile NOD umfassen anorganische Nitrite, Alkylnitrite wie Isopentylnitrit, Diazeniumdiolate (z.B. US Patente Nr. 7,105,502; 7,122,529; 6,673,338), trans[RuCl([15]aneN4)NO]$^{2+}$, Nitrosyl-Liganden, 6-Nitrobenzo[a]pyrol, S-Nitroso-Glutathion, S-Nitroso-Thiole, S-Nitroso-N-Acetyl-D-Penicillamin (SNAP), Nitroanilin-Derivate (siehe US 2013/0224083 A1), 2-Methyl-2-Nitrosopropan, Imidazoyl-Derivate, Nitratester, Hydroxylnitrosamin, Hydroxylamin, Hydroxyharnstoff oder Natrium-Nitroprussid.

In bevorzugter Weise ist der pH-labile NO-Donor ein anorganisches Nitritsalz, das zweckmäßigerweise eine pharmakologisch verträgliche Substanz darstellt. Als solche kommen beispielsweise Nitrite von Alkali- oder Erdalkalimetallen zur Anwendung. Beispielhaft sind hier genannt: $LiNO_2$, $NaNO_2$, $KNO_2$, $RbNO_2$, $CsNO_2$, $FrNO_2$, $Be(NO_2)_2$, $Mg(NO_2)_2$, $Ca(NO_2)_2$, $Sr(NO_2)_2$, $Ba(NO_2)_2$, oder $Ra(NO_2)_2$ und Kombinationen hiervon.

Besonders bevorzugt ist hierbei als NOD das $NaNO_2$, das in weiterhin bevorzugter Weise zusammen mit einer Kombination aus Ascorbinsäure und Trolox als Antioxidantien in der Badelösung eingesetzt wird.

Die Konzentration der Nitritsalze bezogen auf das Gesamtgewicht der sie enthaltenden Badelösung kann

hierbei bis zu 20 Gew.-% betragen, bevorzugt zwischen 0,25 und 10 Gew.-%, besonders bevorzugt zwischen 3 und 7,5 Gew.-%.

In einer alternativen Ausgestaltung kann auch ein Nitratsalz verwendet werden, bei denen eine enzymatische Umwandlung in das entsprechende Nitritsalz möglich ist. Bevorzugt sind hierbei Nitrate von Alkali- oder Erdalkalimetallen zur Anwendung. Beispielhaft sind hier genannt: $LiNO_3$, $NaNO_3$, $KNO_3$, $RbNO_3$, $CsNO_3$, $FrNO_3$, $Be(NO_2)_3$, $Mg(NO_2)_3$, $Ca(NO_2)_3$ $Sr(NO_2)_3$, $Ba(NO_2)_3$, oder $Ra(NO_2)_3$. Die Konzentration der Nitratsalze bezogen auf das Gesamtgewicht der sie enthaltenden Badelösung kann hierbei bis zu 20 Gew.-% betragen, bevorzugt zwischen 0,25 und 10 Gew.-%, besonders bevorzugt zwischen 3 und 7,5 Gew.-%.

Antioxidans

Um die bei der NO-Generierung auftretenden mehrfach oxidierten Stickoxide, Sauerstoffradikalanionen oder Hydroxylradikale zu entfernen, ist es notwendig, dass die Badelösung mindestens ein Antioxidans umfasst.

Nach Art des chemischen Wirkmechanismus werden Antioxidantien in Radikalfänger oder Reduktionsmittel unterschieden.

Bei Oxidationsreaktionen zwischen organischen Verbindungen treten vielfach kettenartige Radikalübertragungen auf. Hier werden Stoffe mit sterisch behinderten Phenolgruppen wirksam, die im Ablauf dieser Übertragungen reaktionsträge, stabile Radikale bilden, die nicht weiter reagieren, wodurch es zum Abbruch der Reaktionskaskade kommt (Radikalfänger). Zu ihnen zählen natürliche Stoffe wie die Tocopherole und synthetische wie Butylhydroxyanisol (BHA), Butylhydroxytoluol (BHT) und die Gallate. Sie sind insbesondere bei unpolaren Flüssigkeiten als Badelösung anzuwenden.

Des Weiteren können auch Reduktionsmittel mit einem sehr niedrigen Standard-RedoxPotential von weniger als + 0,4 V (bei pH 7,0 und 25°C) eingesetzt werden. Typische Vertreter sind etwa Ascorbinsäure (-0,04 V bei pH 7 und 25°C), Salze der Schwefligen Säure (+0,12 V bei pH 7 und 25 °C) und bestimmte organische schwefelhaltige Verbindungen (z. B. Glutathion, Cystein, Thiomilchsäure), die vorwiegend in wässrigen Badelösungen als Trägermedien eingesetzt werden können.

In einer bevorzugten Ausführungsform ist das mindestens eine Antioxidans in der Lage das als NO-Donor im sauren Milieu vorliegende $HNO_2$ zu NO zu reduzieren. Hierzu muss das Antioxidans als Reduktionsmittel ein Standard-Redoxpotential von weniger als +1,0362 Volt, bevorzugt von weniger als + 0,5 Volt, besonders bevorzugt von weniger als + 0,2 Volt und insbesondere bevorzugt von weniger als 0 Volt aufweisen.

Das mindestens eine Antioxidans ist zweckmäßigerweise in der Lage das schädliche $NO_2$-Radikal zum $NO_2^-$-Anion zu reduzieren. Für eine effektive Eliminierung des $NO_2$ Radikals sollte das mindestens eine Antioxidans bevorzugt eine bimolekulare Reaktionskonstante k aufweisen, die größer als $1{,}0 \times 10^6\ M^{-1}s^{-1}$ und bevorzugt größer als $1{,}0 \times 10^7\ M^{-1}s^{-1}$ ist. Erfindungsgemäß geeignete Antioxidantien mit den dazugehörigen Reaktionskonstanten sind in Kirsch et al., 2002 (Biol. Chem 383; 389 -399, s. Tabelle 1) offenbart. Beispielhaft seien hier genannt: Captoprilthiolat, Kaffeesäure, Sinapinsäure, Ferulasäure, Lycopen, Zeaxanthin, Lutein, Astaxanthin, Canthaxanthin, Arachidonat, Gly-Tyr-Dipeptid, Tyrosin, Purine und Pyrimidine wie die Nucleobasen Adenin, Guanin, Cytosin, Thymin, Uracil und die entsprechenden Derivate und Analoga hiervon inklusive der sie enthaltenden Nucleoside und Nucleotide.

In einer weiteren Ausführungsform enthält die erfindungsgemäß verwendete Badelösung, die in bevorzugterweise eine wässrige Flüssigkeit ist, neben dem Antioxidans auch einen Antioxidationssynergisten. Synergisten unterstützen die Wirkung von Antioxidantien, indem sie beispielsweise verbrauchte Antioxidantien wieder regenerieren (sog. "Redox cycling"). Durch Komplexierung von Metallspuren (Natrium-EDTA) oder Schaffung eines oxidationshemmenden pH-Wertes können Synergisten die antioxidative Wirkung eines Radikalfängers oder Reduktionsmittels verstärken. Typische Beispiele für Antioxidantionssysnergisten sind EDTA, 1-Hydroxyethan-1.1-diphosphonsäure, Citronensäure, Fumarsäure, Harnsäure und 2-(Hydroxymethyl)-1,4-benzyldiol.

Bei dem erfindungsgemäßen Herstellungsverfahren wird besonders bevorzugt das Ascorbat oder die Ascorbinsäure als Antioxidans eingesetzt.

Dem Fachmann sind zahlreiche Antioxidantien bekannt, welche in der Lage sind, mehrfach oxidierte Stickoxide, Sauerstoffradikalanionen, Hydroxylradikale oder aquatisierte Elektronen abzubauen oder zu neutralisieren. Diese wird er entsprechend der jeweiligen Zusammensetzung der Badelösung auswählen.

Für apolare Badelösungen eignen sich beispielsweise Antioxidantien wie Tocopherole, Tocotrienole, Tocomonoenole, Irganox®, Irgafos®, Butylhydroxyanisol (BHA) und Butylhydroxytoluol (BHT).

Für polare Badelösungen, wie bspw. wässrige Flüssigkeiten eignen sich wasserlösliche Vitamin E-Derivate wie Trolox oder alpha-AMG, organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure, Ferulasäure, Sinapinsäure oder Kaffeesäure, oder Hydroxybenzoesäuren wie Gallussäure, Procatechusäure, Syringasäure oder Vanillinsäure.

Andere bevorzugte Antioxidantien umfassen polyphenolische Verbindungen wie Anthocyane, Flavonoide und

Phytoöstrogene.

In einer bevorzugten Ausführungsform ist das mindestens eine Antioxidans aus Schritt (a) oder (b) ein Gemisch aus einem Vertreter der Reduktongruppe und einem Vertreter der 6-Hydroxy-Chromangruppe oder der Thiole. Es hat sich erfindungsgemäß herausgestellt, dass eine solche Antioxidantien-Kombination die bei der Reaktion entstehenden schädlichen Radikale besonders wirksam eliminieren kann, ohne dass die NO-Bildung hierdurch beeinträchtigt wird.

In einer bevorzugten Ausführungsform wird im Schritt (a) oder (b) eine Antioxidantien-Kombination gemäß der folgenden Tabelle eingesetzt. Die vorteilhafte Verwendung dieser Kombinationen beruht auf der Tatsache, das ein erstes Antioxidans bevorzugt das $HNO_2$ reduziert (Antioxidans I) und ein zweites Antioxidans bevorzugt das schädliche $NO_2$-Radikal abfängt (Antioxidans II). Die Tabelle stellt einerseits die allgemeine Stoffklasse vor, um dann beispielhaft einige bevorzugte konkrete Substanzkombinationen zu offenbaren.

| Antioxidans I | Antioxidans II |
|---|---|
| **Redukton** | **6-Hydroxychroman** |
| - Ascorbinsäure | - Trolox |
| - Isoascorbinsäure | - Trolox |
| - Erythroascorbinsäure | - Trolox |
| - Ascorbylstearat | - alpha-Tocopherol |
| - Ascorbylpalmitat | - alpha-Tocopherol |
| **Redukton** | **Thiol** |
| - Ascorbinsäure | - Cystein |
| - Isoascorbinsäure | - Cystein |
| - Erythroascorbinsäure | - Cystein |
| - Ascorbylstearat | - Cystein |
| - Ascorbylpalmitat | - Cystein |
| - Ascorbinsäure | - Glutathion |
| - Isoascorbinsäure | - Glutathion |
| - Erythroascorbinsäure | - Glutathion |
| - Ascorbylstearat | - Glutathion |
| - Ascorbylpalmitat | - Glutathion |

Erfindungsgemäß ist unter einem Vertreter der Reduktongruppe eine organisch-chemische Verbindung zu verstehen, die an den beiden Kohlenstoffatomen einer C=C-Doppelbindung zwei Hydroxylgruppen trägt ("Endiol") sowie zusätzlich direkt am benachbarten Kohlenstoffatom eine Carbonylgruppe aufweist. Die Doppelbindung dieser Endiole ist wegen der Konjugation mit der Carbonylgruppe stabilisiert; daher liegt im tautomeren Gleichgewicht ("Keto-Enol-Tautomerie") hauptsächlich die Endiolform und nicht die Ketoform vor. Als vinyloge Carbonsäuren reagieren Reduktone sauer. Die Reduktongruppe umfasst beispielweise Ascorbat und Derivate hiervon, Hydroxypropandial (Tartronaldehyd), trans-3,4-Dihydroxy-3-hexen-2,5-dion (DHHD) und 2,3-Dihydroxy-2-cyclopentenon (Reduktinsäure). In bevorzugter Weise wird als Vertreter der Reduktongruppe die Ascorbinsäure oder das Ascorbat und Derivate hiervon wie Erythroascorbinsäure oder Ascorbylpalmitat eingesetzt.

Vertreter der 6-Hydroxychroman-Gruppe sind erfindungsgemäß Substanzen, die einen in 6-Position hydroxylierten Chromanring umfassen, der darüber hinaus an den anderen Positionen statt Wasserstoff einen oder mehrere weitere (bevorzugt Methyl)-Substituenten tragen kann. Typische Vertreter der 6-Hydroxychroman-Gruppe sind Tocopherole. Tocomonoenole und Tocotrienole und Derivate hiervon wie beispielsweise (RS)-6-Hydroxy-2,5,7,8-tetramethylchroman-2-carbonsäure (Trolox). In bevorzugter Weise wird als 6-Hydroxychroman-Vertreter das alpha-Tocopherol oder Trolox eingesetzt.

Thiole (auch Thioalkohole genannt) sind gemäß der Erfindung organisch-chemische Verbindungen, die eine oder mehrere aliphatisch oder aromatisch gebundene Thiolgruppen (-SH) als funktionelle Gruppen tragen. Erfindungsgemäß sind Cystein und Glutathion als Thiole bevorzugt.

Die Endkonzentration der Thiole in der Badelösung liegt hierbei bevorzugt zwischen 1 und 1000 mM, besonders

bevorzugt zwischen 20 und 200 mM und insbesondere bevorzugt zwischen 50 und 100 mM.

Für ein polare Badelösung, wie bspw. ein wässrige Flüssigkeit, können zweckmäßigerweise wasserlösliche Vertreter der vorabgenannten Gruppen kombiniert werden, also beispielsweise Ascorbat und (RS)-6-Hydroxy-2,5,7,8-tetramethylchroman-2-carbonsäure (Trolox), Ascorbat und Cystein, oder in bevorzugter Weise Ascorbat und N-Acetylcystein.

Für eine unpolare Badelösung kann man zwei lipophile Vertreter der vorabgenannten Gruppen einsetzen, also beispielsweise Ascorbylpalmitat, Ascorbylstearat und alpha-Tocopherol einsetzen und bevorzugt eine Kombination aus Ascorbylpalmitat und alpha-Tocopherol oder Ascorbylstearat und alpha-Tocopherol.

Zweckmäßigerweise liegt das mindestens eine Antioxidans im Verhältnis zu dem NO-Donor in einem molaren Überschuss vor.

Bei einer Kombination aus zwei Antioxidantien mit $HNO_2$ und $NO_2$-Radikal Reaktionspräferenz (im Rahmen der Erfindung als Antioxidans I und Antioxidans II bezeichnet) ist es vorteilhaft, wenn sie eine einem molaren Verhältnis gemäß der folgenden Formel vorliegen:

$$\text{mol[NO-Donor]} < \text{mol [Antioxidans I]} < \text{mol[Antioxidans II]}.$$

Da die Eliminierung von $NO_2$-Radikalen eine besonders wichtige Aufgabe gerade im Bereich der therapeutischen und kosmetischen Anwendung darstellt, sollte das Antioxidans II aus Sicherheitserwägungen in einem größeren molaren Verhältnis vorliegen.

In bevorzugter Weise enthält die Badelösung in Schritt (a) oder (b) die drei Komponenten: NO-Donor, Antioxidans I und Antioxidans II in einem molaren Verhältnis von 1 : 2-20 : 4-100, wobei das molare Verhältnis: Nitrit < Ascorbat < Trolox ist. Bevorzugt ist hier ein molares Verhältnis von 1 : 2-10 : 5-50, besonders bevorzugt von 1 : 3-8 : 5-20 und speziell ein Verhältnis von 1: 5 : 10.

In einer Ausführungsform der Erfindung enthält die Badelösung und insbesondere in ihrer Ausgestaltung als wässrige Flüssigkeit zusätzlich einen oder mehrere der folgenden Stoffe: Katalysatoren, Detergentien, Puffersubstanzen, Chromophore, Substanzen, die das Prodrug stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung der Badelösung zur topischen Anwendung physiologische verträgliche und/oder dermatologisch verträgliche Stoffe und Stoffgemische zur Anwendung kommen.

<u>Säureaktivierung im Schritt (b)</u>

Für die Spaltung des pH-labilen NO-Donors wird die Flüssigkeit auf einen sauren pH-Wert gebracht. Dieser pH-Wert liegt hierbei erfindungsgemäß so niedrig, dass er die Spaltung des pH-labilen NO-Donors unter Bildung von NO induziert. Der konkrete pH-Wert hängt von der pH-Labilität des NO-Donors und der erwünschten Zeitspanne für die NO-Generierung ab. Je geringer der pH-Wert, desto schneller wird in der Badelösung das NO erzeugt werden.

Gemäß der Erfindung beträgt der pH-Wert im Schritt (b) hierbei zwischen 0,0 und 6,9, bevorzugt zwischen 2,0 und 6,0, besonders bevorzugt zwischen 4,5 und 6,0 und insbesondere bei 50. Der optimale Wert für den pH-Wert ist wie oben bereits ausgeführt von dem jeweils verwendeten NO-Donor und der intendierten Reaktionsgeschwindigkeit abhängig und wird vom Fachmann entsprechend eingestellt werden.

In einer Ausführungsform der Erfindung wird bei dem Verfahren das für die NO-Freisetzung aus dem pH-labilen NO-Donor notwendige saure Milieu durch Zugabe einer Säure oder einen Puffers mit saurem pH-Wert (d.h. mit pH<7) erzeugt.

Als Säuren stehen dem Fachmann hierzu zahlreiche Säuren zur Verfügung. Dies umfasst sowohl Mineralsäuren wie HCl, $H_2SO_4$, $H_3PO_4$ oder $HNO_3$, als auch organische Säuren wie Essigsäure, Zitronensäure oder Milchsäure.

In einer besonderen Ausführungsform ist die Säure gleichzeitig ein Antioxidans, wie beispielsweise Ascorbinsäure oder Thiomilchsäure oder ein Antioxidationssynergist wie 1-Hydroxyethan-1.1-diphosphonsäure oder Harnsäure. Hierdurch kann auf die Anwesenheit eines Antioxidans im Schritt (a) verzichtet werden. Das Antioxidans wird als Säure im Schritt (b) hinzugegeben und kommt so gezielt ab dem Zeitpunkt zum Einsatz, ab dem durch die Spaltung des NO-Donors schädliche oder unerwünschte Radikale auftreten.

In einer weiteren Ausführungsform liegt die Säure in fester Form vor und wird durch Zusammenbringen mit der Badelösung gelöst und damit deprotonierbar. Hierbei kann die Säure in Form von Pulver, Granulat, Nanopartikeln oder als an einem Polymer befindliche Säuregruppe vorliegen.

Photolatente Säure

In einer bevorzugten Ausführungsform der Erfindung wird die NO-Generierung im Schritt (b) durch Aktivierung eine photolatenten Säure initiiert, die durch Bestrahlung mit der elektromagnetischen Strahlung die Säure freisetzt, also zu einer Ansäuerung der Flüssigkeit führt. Dies hat den Vorteil, dass der Reaktion keine Säure von außen zugefügt werden muss, sondern die Ansäuerung durch einer in der Badelösung vorhandenen Substanz induziert werden kann.
Diese Ausführungsform ist insbesondere vorteilhaft, wenn im Schritt (e) weiteres NO durch einen photolytischen Prozess generiert wird, da dann schon die Lichtquelle bei der erfindungsgemäßen Badevorrichtung vorgesehen ist.
Zudem ist es hier vorteilhaft, dass die Bestrahlung als initiales Ereignis eine länger anhaltende NO-Freisetzung induzieren kann und somit wie ein "Schalter" wirkt, der die erfindungsgemäße NO-Erzeugung startet.
Beispiele für photolatente Säuren sind z.B. Oniumsalze, wie Sulfonium- oder lodoniumsalze, sowie Oximsulfonsäureester. Solche Verbindungen sind in der Technik bekannt und in einer Vielzahl in der Literatur beschrieben.
Beispiele sind Triarylsulfonium- oder Diaryliodoniumsalze, z.B. unsubstituiert oder mit Alkyl- oder Alkoxysubstituenten mit den unterschiedlichsten Anionen, wie beispielsweise $HSO_4^-$, $PF_6^-$, SbF6$^-$, $AsF_6^-$, $Cl^-$, Br, $I^-$, $ClO_4^-$, $PO_4^-$, $SO_3CF_3^-$, Tosylat, oder einem Borat-Anion, wie etwa $BF_4^-$, oder $B(C_6F_5)_4^-$.
Oniumsalze sind z.B. von J.V. Crivello, K. Dietliker "Photoinitiators for Free Radical, Cationic & Anionic Photopolymerisation", Band III von "Chemistry & Technology of UV & EB Formulation for Coatings, Inks & Paints", 2. Ed., J. Wiley and Sons/SITA Technology (London), 1998 (insbesondere Seiten 464-466) beschrieben. lodoniumsalze sind aus einer Vielzahl von Patentschriften bekannt, z.B. US 4151175, US 3862333, US 4694029, EP 562897, US 4399071, WO 98/46647 usw., und sind beispielsweise "symmetrische" oder "unsymmetrische" Diarylјodoniumverbindungen der Formel (C)

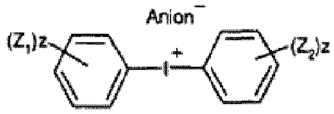

(C),

worin
$Z_1$ und $Z_2$ gleich oder verschieden sind und beispielsweise lineares oder verzweigtes $C_1$-$C_{20}$-Alkyl, $C_1$-$C_{20}$-Alkoxy, Halogen, $C_2$-$C_{12}$-Alkenyl, Cycloalkyl darstellen; und z unabhängig voneinander für 0 bis 5, insbesondere für 0 oder 1, stehen, d.h. für den Fall, dass mehrere Reste $Z_1$ oder $Z_2$ vorliegen, z also grösser als 0 ist, alle $Z_1$ oder alle $Z_2$ nicht die gleiche Bedeutung haben müssen.
Weitere photolatente Säurespender sind von M. Shirai und M. Tsunooka in Prog. Polym. Sci., Vol. 21, 1-45 (1996), in Form einer Übersicht zusammengefasst.
Andere geeignete photolatente Säuren sind Oximsulfonate. Auch diese Verbindungen sind in der Technik bekannt und beispielsweise in US 5237059, EP 571330, EP 241423, EP 139609, EP 361907, EP 199672, EP 48615, EP 12158, EP 780729 offenbart.
Beispiele sind α-(Methylsulfonyloxyimino)-4-methoxybenzylcyanid, α-(Methylsulfonyloxyimino)-3-methoxybenzylcyanid, α-(Methylsulfonyloxyimino)-3,4-dimethylbenzylcyanid, α-(Methylsulfonyloxyimino)-thiophen-3-acetonitril, α-(Isopropylsulfonyloxyimino)-thiophen-2-acetonitril, cis/trans-α-(Dodecylsulfonyloxy-imino)-thiophen-2-acetonitril, ESACURE (Lamberti), IRGACURE (Ciba) e.g. IRGACURE® PAG103 (2-Methyl-α-[2-[[[(n-propyl)sulfonyl]oxy]imino]-3(2H)-thienyliden]-benzylacetonitril, 2(5H)-Thienyliden]-Benzylacetonitril), IRGACURE® PAG108 (2-Methyl-α-[2-[[[(n-octyl)sulfonyl]oxy]imino]-3(2H)-thienyliden]-benzyl-acetonitril), IRGACURE® PAG121 (2-Methyl-α-[2-[[[(4-Methylphenyl)sulfonyl] oxy]imino]-3(2H)-thienyliden]-benzylacetonitril), IRGACURE® PAG203, Ethanon, 1,1'-[1,3-propandiylbis(oxy-4,1-phenylen)] bis-[2,2,2-trifluoro-bis[O-(propylsulfonyl) oxim], UVI (DOW Chemicals), CYRACURE (DOW Chemicals), und 2-(-Methoxystyryl)-4,6-bis(trichloro-methyl)-1,3,5-triazin (Sigma Aldrich).
Geeignet sind z.B. auch die in WO 2000/1097 A2 oder GB 2348644 beschriebenen Oximsulfonate. Oximverbindungen, welche andere Säuren als Sulfonsäuren abgeben sind ebenfalls geeignet und beispielsweise in WO 00/26219 offenbart.
Die vorstehende Aufzählung ist im Zusammenhang der vorliegenden Erfindung lediglich als beispielhaft und keineswegs als limitierend zu verstehen.
Erfindungsgemäß sind hier photolatente Lewissäuren bevorzugt. Bei der photolatenten Lewissäure handelt es sich um eine photochemisch aktive Substanz, das heißt, um eine Substanz, die in der Lage ist, aus eingestrahltem Licht derart Energie aufzunehmen, dass diese Substanz infolge der Energieaufnahme in einer chemischen Reaktion verändert wird und dabei eine Lewissäure freisetzt.
Dazu weist die photolatente Lewissäure bei den Wellenlängen des einstrahlenden Lichts, deren Dosis jeweils zu überwachen ist, eine von Null verschiedene Absorption auf, so dass die Strahlung vollständig oder zumindest teilweise von der photolatenten Lewissäure absorbiert wird und diese in einen energetisch angeregten Zustand überführt. Der

energetisch angeregte Zustand hat die Freisetzung der Lewissäure zur Folge. Hierdurch wird die Konzentration an freier Lewissäure in der Badelösung lokal erhöht, was in einer säureinduzierten Spaltung des pH-labilen NO-Donors resultiert.

Als photolatente Lewissäure kommt grundsätzlich jede Substanz in Frage, die zumindest in einem Wellenlängenbereich der Strahlung eine von Null verschiedene Absorption aufweist und die darüber hinaus in der Lage ist, infolge der Absorption der Strahlung eine Lewissäure freizusetzen, das heißt in einer chemischen Reaktion zu erzeugen oder anderweitig als freie Verbindung zur Verfügung zu stellen, beispielsweise in einem Desorptionsschritt oder aus einem Lewisaddukt. Bei der Lewissäure kann es sich beispielsweise um einen von der photolatenten Lewissäure abgespaltenen Teil handeln. Als Lewissäuren werden alle elektrophilen Elektronenpaarakzeptoren verstanden, also alle Substanzen, die Elektronenpaare anlagern können, beispielsweise Moleküle und Ionen mit unvollständiger Edelgaskonfiguration, also einer Elektronenlücke.

Insbesondere gelten als Lewissäuren im Sinne dieser Erfindung auch Bronstedsäuren (klassische Säuren; Protonensäuren), das heißt Substanzen, die Protonendonatoren sind oder diese enthalten, wobei hierunter auch Protonen selber zu verstehen sind.

Beispiele für erfindungsgemäß einsetzbare photolatente Lewissäuren sind etwa aus WO 02/101462 A1 und WO 2005/097876 A1 bekannt, auf die hier ausdrücklich Bezug genommen wird.

Als latente Lewissäuren kommen gemäß WO 2005/097876 A1 insbesondere solche in Frage, die auf einer Verbindung der allgemeinen Formel $R^1$-CH*$R^0$-($A^6$)$R^2R^3R^4R^5$-OH basieren. Dabei stellt $A^6$ ein aromatisches Ringsystem mit sechs Ringatomen dar, das optional ein Heteroatom oder mehrere Heteroatome und/oder weitere annellierte Ringe enthalten kann. $R^1$ wird gewählt aus der Gruppe umfassend Wasserstoff, Alkylgruppen (insbesondere $C_1$-$C_{20}$-Alkylgruppen), Alkenylgruppen (insbesondere $C_2$-$C_{20}$-Alkenylgruppen), Arylgruppen (insbesondere unsubstituierte sowie einfach, zweifach oder dreifach mit $C_1$-$C_4$-Alkylgruppen substituierte Phenylgruppen, oder $C_1$-$C_4$-Alkoxygruppen. $R^2$, $R^3$, $R^4$ sowie $R^5$ werden unabhängig voneinander gewählt aus der Gruppe umfassend Wasserstoff oder funktionale Substituenten. R° wird gewählt aus der Gruppe umfassend $C_1$-$C_6$-Alkylgruppen, oder Gruppen der allgemeinen Formel -$Z^1$-$Q^1$ oder -$Z^2$-$Q^2$. $Z^1$ stellt dabei eine Einfachbindung dar oder ein verbrückendes Schwefelatom (-S-) oder Sauerstoffatom (-O-) oder eine verbrückende sekundäre Amingruppe (-NH-). $Q^1$ stellt dabei ein heterocyclisches Ringsystem mit 5 bis 9 Ringatomen dar, dessen Ringatome Kohlenstoff (C), Schwefel (S), Sauerstoff (O) und Stickstoff (N) sein können, wobei das Ringsystem zumindest zwei, bevorzugt drei, besonders bevorzugt wenigstens vier Kohlenstoffatome enthält. Insbesondere repräsentiert $Q^1$ Morpholin, Pyridin (das gegebenenfalls einfach bis dreifach mit C1-C2-Alkylgruppen oder Hydroxylgruppen substituiert sein kann), Mercaptobenzoxazol oder Mercaptobenzthiazol. $Z^2$ steht für eine $C_1$-$C_4$-Alkylengruppe, die mit einer $C_1$-$C_4$-Alkylgruppe oder mit $Q^3$ substituiert sein kann. $Q^2$ und $Q^3$ stellen hierbei unabhängig voneinander Phenylgruppen dar, die gegebenenfalls einfach bis dreifach mit $C_1$-$C_4$-Alkylgruppen, Hydroxylgruppen, $C_5$-$C_8$-Cycloalkylgruppen und/oder einem heterocyclischen Ringsystem mit 5 bis 9 Ringatomen substituiert sein können, dessen Ringatome Kohlenstoff (C), Schwefel (S), Sauerstoff (O) und Stickstoff (N) sein können, wobei das Ringsystem zumindest zwei, bevorzugt drei, besonders bevorzugt wenigstens vier Kohlenstoffatome enthält. Darüber hinaus kann das Wasserstoffatom H*, das an dem Kohlenstoffatom in Bezug auf den Substituenten R" in alpha-Stellung gebunden ist, bei Einwirkung von elektromagnetischer Strahlung in einer photochemischen Reaktion als Proton abgespalten werden.

Spezifische Beispiele für photolatente Lewissäuren sind in WO 02/101462 A1 beschrieben, die, ohne sich durch diese Beispiele einzuschränken, ausnahmslos eingesetzt werden können.

Als photolatente Säuren können ebenfalls die in WO 2003/050912 beschriebenen phenolischen Antioxidantien eingesetzt werden. Typische Beispiele hierfür sind etwa Verbindungen aus der Gruppe der Hydroxyphenylbenzotriazole, der Hydroxyphenyltriazine oder der Hydroxybenzophenone, die allesamt eine Hydroxylgruppe aufweisen, die an einem Phenylring in Bezug auf die Bindung zwischen dem Phenylring und dem Molekülhauptgerüst in ortho-Stellung angeordnet sind.

In einer Ausführungsform der Erfindung weist der Schritt (c), der die NO-Generierung beinhaltet, eine Zeitdauer von zwischen 15 Sekunden und 1 Stunde, bevorzugt von zwischen 1 und 30 Minuten, besonders bevorzugt von zwischen 5 und 20 Minuten und besonders bevorzugt von zwischen 10 und 15 Minuten auf.

Bei der Badelösung liegt die Konzentration des gebildeten NO zwischen 0,01 und 2 mM, bevorzugt zwischen 0,05 bis 1 mM und besonders bevorzugt zwischen 0,1 und 0,5 mM.

## pH-Werterhöhung

In einem der primären NO-Generierung gemäß Schritt (c) nachgeschaltetem Verfahrensschritt (d) wird der pH-Wert der Flüssigkeit erhöht. Diese pH-Werterhöhung kann erfindungsgemäß durch Zugabe einer Base, eines basischen Puffersystems oder durch Photoaktivierung einer photolatenten Base geschehen.

Die erfindungsgemäße pH-Wert-Erhöhung weist erfindungsgemäß eine oder mehrere der folgenden Eigenschaften auf:

(a) eine Erhöhung des pH-Wertes auf pH 7,0 oder mehr;
(b) eine Erhöhung des pH-Werts um mindestens eine pH-Wertstufe;
(c) eine Erhöhung des pH-Werts auf einen pH-Wert, der mit einer reduzierten NO-Generierung einhergeht, so dass die Menge an neugebildetem NO der Menge des in der Badelösung abnehmenden NOs entspricht.

Der pH-Wert wird erfindungsgemäß so stark erhöht, dass die Säure-induzierte NO-Generierung stark inhibiert wird oder sogar vollständig unterbleibt oder in einer besonderen Ausführungsform die NO-Generierung in einem reduziertem Maße derart erlaubt, dass diese Neubildung die Konzentrationsabnahme an NO (sei sie durch Zerfall, Abreaktion oder Freisetzung bedingt) kompensiert. In diesem Sinne sorgt die pH-Werterhöhung für die Aufrechterhaltung eines Fließgleichgewichts an NO.

**[0181]** Zweckmäßigerweise führt die pH-Werterhöhung in Schritt (d) hierbei zu einem pH-Wert, der zwischen 7,0 und 12,0, bevorzugt zwischen 7,0 und 9,0, besonders bevorzugt zwischen 7,0 und 8,0 und insbesondere bei 7,5 liegt.

Als Basen stehen dem Fachmann hierzu zahlreiche Basen zur Verfügung. Dies umfasst sowohl anorganische Basen wie $NH_4OH$ als auch organische Basen wie aliphatische oder aromatische Amine.

In einer Ausführungsform wird zur pH-Werterhöhung eine Base verwendet wird, die ausgewählt ist aus der Gruppe enthaltend NaOH, KOH, $Ca(OH)_2$, $NH_4OH$ und Natriumhydrogencarbonat.

In einer alternativen Ausführungsform wird zur pH-Werterhöhung ein basischer Puffer verwendet, der ausgewählt ist aus der Gruppe enthaltend Phosphatpuffer, Barbital-Acetatpuffer, 4-(2-Hydroxyethyl)-1-piperazinethanesulfonsäure (HEPES)-Puffer, Tris(hydroxymethyl)-aminomethan(TRIS)-Puffer, 4-(2-Hydroxyethyl)-piperazin-1 -propansulfonsäure (HEPPS)-Puffer, Barbital-Acetat-Puffer, Essigsäure-Acetat-Puffer, Kohlensäure-Silicat-Puffer, 2-(N-Morpholino)ethansulfonsäure (MES)-Puffer, Kohlensäure-Bicarbonat-Puffer, Citronensäure-Puffer oder Citrat-Puffer.

<u>Photolatente Basen</u>

In einer bevorzugten Ausführungsform wird für die pH-Werterhöhung eine photolatente Base verwendet, die durch Bestrahlung mit der elektromagnetischen Strahlung die Base freisetzt, also zu einer pH-Werterhöhung der Badelösung, die bevorzugt eine wässrige Flüssigkeit ist, führt. Eine solche photolatente Base hat den Vorteil, dass auch hier keine Base extern dem System hinzugefügt werden muss, sondern dass auch hier die erfindungsgemäß optional eingesetzte (UV)-Lichtquelle den pH-Wert-Shift von außen auslösen kann.

Beispiele für photolatente Basen sind z.B. $\alpha$-Aminoacetophenone, Oniumsalze wie Sulfonium- oder Iodoniumsalze, sowie Oximsulfonsäureester. Solche Verbindungen sind in der Technik bekannt und in einer Vielzahl in der Literatur beschrieben.

Beispiele für erfindungsgemäß einsetzbare photolatente Basen sind etwa aus EP 0 898 202 A1, WO 94/28075 A1, WO 01/92362 A1, EP 0 970 085 A1 und WO 03/033500 A1 bekannt, auf die hier ausdrücklich Bezug genommen wird.

Geeignete photolatente Basen umfassen N-substitutierte 4-(o-Nitrophenyl) dihydropyridine, optional substitutiert mit Alkylether und/oder Alkylestergruppen, und quaternäre organische Bor-Photoinitiatoren. Beispiele für N-substitutierte 4-(o-Nitrophenyl)dihydropyridine sind N-Methyl-Nifedipin, N-Butyl-Nifedipin, N-butyl 2,6-dimethyl 4-(2-nitrophenyl)1,4-dihydropyridin 3,5-dicarboxylsäurediethylester und ein Nifedipin gemäß der folgenden Formel:

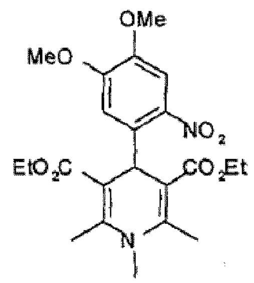

i.e., N-Methyl 2,6-Dimethyl 4-(4,5-Dimethoxy-2-Nitrophenyl)1,4-Dihydropyridin 3,5-dicarbonsäurediethylester. Beispiele für Organo-Bor-Verbindungen sind in der GB-A-2 307 473 offenbart, wie beispielsweise

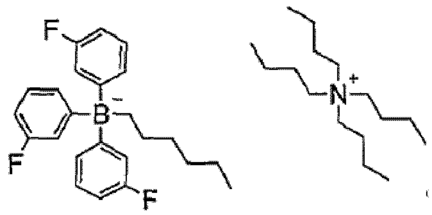

Aus dem Stand der Technik sind insbesondere die α-Amino-Acetophenon-Derivate als effiziente photolatente Basen bekannt. Beispiele für α-Amino-Acetophenone, die in dem erfindungsgemäßen Verfahren eingesetzt werden können, sind: 4-(Methylthiobenzoyl)-1-Methyl-1-Morpholinoethan (Irgacure®907ex, Ciba Spezialchemie) und (4-Morpholinobenzoyl)-1-benzyl-1-dimethylaminopropan (Irgacure®369ex, Ciba Spezialchemie), die auch in der EP 0 898 202 A1 offenbart werden. Bevorzugt ist ein α-Amino-Acetophenon der folgenden Formel:

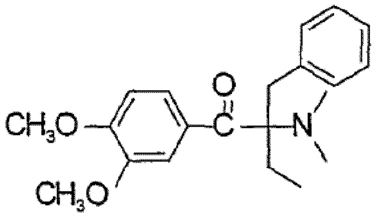

Die WO 94/28075 beschreibt UV-entblockbare Basen vom Amin, Ammonium oder Phosphan-Typ. Als blockierende Agenzien werden insbesondere alpha-Ketocarboxylsäuren, aromatische oder N-heterozyklische Ameisen-, Essig- oder Glyoxylsäurederivate, mit denen die Basen in ihre nicht reaktiven Salze umgewandelt werden und die durch Bestrahlung deblockiert werden können. Die WO 97/31033 beschreibt die photochemische Freisetzung von Basen mit einem $pK_a$ ~12, beispielhaft sei hier das N-Benzyloxycarbonyltetramethylguanidin erwähnt. Ionische Salze von α-Ammonium, α-Iminium oder α-Amidiniumketonen oder Alkenen, die bei Bestrahlung die korrespondierenden tertiären Aminbasen freisetzen, sind beispielsweise in der WO1998/38195 und der WO 2000/10964 offenbart. Die WO 1998/32756 offenbart α-Aminoketone, die bei Bestrahlung Amidinbasen freisetzen; korrespondierende α-Aminoalkene sind in der WO 1998/41524 offenbart.

Beispiele für geeignete Basen sind unter anderem tertiäre Amine und Amidine, wie Diazabicyclooctan, N-Aikylmorpholine, Tetramethylguanidin (TMG), Diazabicyclononen (DBN), Diazabicycloundecen (DBU) und Imidazol.

Besonders geeignete Amidine sind photolabile Diazabicyclononane, insbesondere 5-Benzyl-1,5-diazabicyclo[4.3.0]nonan, wobei der 5-Benzyl-Rest auch ein- oder mehrfach substituiert sein kann. Geeignete Substituenten am 5-Benzyl-Rest sind beispielsweise Halogenreste, wie Chlor oder Brom, Alkylreste, wie Methyl, Ethyl, oder Propyl, Nitrilreste, Nitrogruppen, Alkoxygruppen, wie Methoxy oder Ethoxy oder an den 5-Benzylrest ankondensierte aromatische Reste, wobei zum Beispiel aus einem 5-(Benzyl)rest ein 5-(Naphth-2-ylmethyl)rest oder ein 5-(Anthracen-9-yl-methyl)rest ableitbar ist. Auch kann anstelle des 5-Benzylrests beispielsweise ein 5-(Anthrachinon-2-yl-methyl)rest treten. Neben den möglichen Substitutionen am 5-Benzyl-Rest kann auch der Diazacyclononanrest weiter substituiert sein, wie zum Beispiel in 5-Benzyl-2-methyl-1,5-diazabicyclo[4.3.0]nonan. Neben den photolabilen Diazabicyclononanen, besteht auch die Möglichkeit photolabile Diazabicycloundecane, wie beispielsweise 8-Benzyl-1,8-diazabicyclo[5.4.0]undecane und dessen Derivate einzusetzen. Der 8-Benzyl-Rest kann analog dem 5-Benzyl-Rest des 5-Benzyl-1,5-diazabicyclo[4.3.0]nonans weiter substituiert sein oder ersetzt werden. Auch hier besteht die Möglichkeit einer weiteren Substitution am Diazabicyclononan-Rest.

Es können auch photolatente Basen eingesetzt werden, die zwei abspaltbare Basen in einem Molekül enthalten. Ein Vertreter dieser Art ist beispielsweise das 1,4-Bis(1,5-Diazabicyclo[4.3.0]nonanylmethyl)benzol. Die Synthese der oben genannten photolatenten Basen ist unter anderem in der WO 03/033500 A1 beschrieben.

## Pharmakologische aktive Substanzen

In einer Ausführungsform der Erfindung enthält die Badelösung und insbesondere in ihrer Ausgestaltung als wässrige Flüssigkeit eine oder mehrere pharmakologisch aktive Substanzen. Diese können die pharmakologische Wirkung des NOs unterstützen oder unabhängig von dem NO in einer für die entsprechende Erkrankung therapeutisch relevanten Weise wirken.

Beisln einer Ausführungsform der Erfindung enthält die Badelösung und insbesondere in ihrer Ausgestaltung als wässrige Flüssigkeit eine oder mehrere der folgenden pharmakologisch aktiven Substanzen: Entzündungshemmer wie bspw. nichtsteroidale Antirheumatika (NSAIDs) oder Corticoide, Immunsuppressiva, Antibiotika, Antikoagulantien,

Antithrombotika, antivirale Agenzien, Antimykotika, Lokalanästhetika und Analgetika.

Optionale Zugabe eines weiteren Antioxidans

In einer bevorzugten Ausführungsform der Erfindung wird mit der pH-Werterhöhung in Schritt (d) oder in einem nachfolgenden Schritt (e) mindestens ein Antioxidans hinzugegeben.

In einer Ausführungsform entspricht dieses mindestens eine Antioxidans dem im Schritt (a) oder (b) bereitgestellten mindestens einen Antioxidans. Auf diese Weise kann das während der NO Generierung verbrauchte Antioxidans wieder durch neues Antioxidans ergänzt werden.

In bevorzugter Weise ist das im Schritt (d) oder(e) neu hinzugegebene, mindestens eine Antioxidans ein Antioxidans, das das vorher zugegebene mindestens eine Antioxidans regenerieren kann. Es wirkt somit als Antioxidationssynergist. Klassischerweise wird das Antioxidans bei der Reduktion der entsprechenden Substanzen selber oxidiert. Für eine Regeneration des Antioxidans muss dieses daher durch ein stärkeres Reduktionsmittel in die reduzierte Form umgewandelt werden (sog. "Redox cycling"). Bei Kenntnis des zu reduzierenden ersten Antioxidans muss der Antioxidationssynergist ein demgegenüber negativeres Standard-Redoxpotential besitzen. Für das bevorzugt verwendete Ascorbat mit eine Redoxpotential von +0,35 Volt ist somit beispielsweise das Cystein mit einem Redoxpotential von - 0,2 Volt (Cystein-Cystin; 25°C, pH 7.0) zur Regeneration geeignet.

In einer bevorzugten Ausführungsform handelt es sich hierbei um ein Antioxidans aus der Stoffklasse der Thiole. Bevorzugte Beispiele hierfür sind: Cystein, Glutathion, N-Acetylcystein, Dimercaptobernsteinsäure, Dimercaptopropansulfonsäure, Ethanthiol (Ethylmercaptan), Dithiothreitol (DTT), Dithioerythritol (DTE), Captopril, Coenzym A, Penicillamin, 1-Propanthiol, 2-Propanthiol, Homocystein, Mesna, Methanthiol (Methylmercaptan) und Thiophenol.

In einer besonderen Ausführungsform liegt ist nach Zugabe eines Thiols als Antioxidans im Schritt (d) die beiden Komponenten NO-Donor, welcher bevorzugt Nitrit ist, und Thiol in einem molaren Verhältnis von 1 : 1-20. Bevorzugt ist hier ein molares Verhältnis von 1 : 2-8, besonders bevorzugt von 1 : 3-7 und speziell ein Verhältnis von 1: 5.

Photolytische NO-Generierunq

In einer weiteren Ausführungsform der Erfindung wird bei dem Verfahren nach Schritt (d) oder (e) die Badelösung zur photolytischen Zersetzung des NO-Donors unter Bildung von NO mit Licht bestrahlt wird. Eine nachgeschaltete photolytische NO-Generierung hat den Vorteil, dass ausgehend von der bereits erzeugten, physiologisch relevanten Menge an NO, eine Abnahme im NO-Gehalt (kumulativ bedingt durch die Weiterreaktion/Zerfall des NOs und die Freisetzung aus der Badelösung) durch die photolytisch-induzierte Neugenerierung in eleganter Weise kompensiert werden kann, das es keiner weiteren Zugabe von Substanzen zur Badelösung bedarf und das Ausmaß der NO-Generierung leicht über die Bestrahlungsdauer und/oder Bestrahlungsintensität steuerbar ist.

Lichtquelle

Erfindungsgemäß kann bei dem Verfahren eine Lichtquelle verwendet werden.

Eine Lichtquelle im Sinne der Erfindung erzeugt dabei eine elektromagnetische Strahlung, die das Spektrum des sichtbaren Lichts, des Infrarotlichts und insbesondere die UV-Strahlung beinhaltet. Die UV-Strahlung umfasst hierbei sowohl die $UV_A$ als auch die $UV_B$-Strahlung.

Die Art der Bestrahlung von NO-generierenden Ausgangssubstraten ist dem auf dem vorliegenden Gebiet tätigen Fachmann an sich bekannt. Es kann jegliche elektromagnetische Strahlung verwendet werden, welche in der Lage ist, photolabile NO-Derivate unter Bildung von Stickstoffmonoxid zu zersetzen. Beispielsweise kann im Rahmen der vorliegenden Erfindung die Herstellung von Stickstoffmonoxid mittels photolytischer Spaltung unter Verwendung einer $UV_A$-Strahlung mit Wellenlängen von beispielsweise 320 bis 400 nm erfolgen. Es kann jedoch auch elektromagnetische Strahlung jeder anderen Wellenlänge verwendet werden, die allein oder unter Zuhilfenahme chemischer, physikalischer oder biologischer Verfahren eine photolytische Spaltung von NO-generierender NO-Vorstufen (NO-Derivate) induziert.

Die Herstellung von Stickstoffmonoxid kann auch in Badelösungen, und hier bevorzugt in wässrigen Flüssigkeiten erfolgen, die mit inerten Gasen gesättigt sind. In solchen mit inerten Gasen (Stickstoff ($N_2$), Helium ($H_2$), Argon, usw.) gesättigten Lösungen hat das darin gelöste NO eine wesentlich längere Lebensdauer und kann auch in höheren Konzentrationen in Lösung verbleiben. Allgemein wird angenommen, dass die maximale Löslichkeit von NO in wässrigen Lösungen ca. 2 mM beträgt. In diesem Zusammenhang können als wässrige Badelösungen auch Kulturmedien oder Infusionsmedien oder Infusionspuffer verstanden werden.

Bei einer Vorrichtung zur Durchführung des erfindungsgemäßen Verfahrens kann die elektromagnetische Strahlung von einer Lichtquelle emittiert werden, die außerhalb und/oder innerhalb der Vorrichtung angebracht sein kann. Wichtig ist, dass die Lichtdurchflutung der Badelösung mitsamt den das Stickstoffmonoxid freisetzenden Reaktionssubstanzen im Sinne eines induzierten Stoffzerfalls bzw. einer Freisetzung von Stickstoffmonoxid maximal ist. Die Quelle der elektromagnetischen Strahlung kann dabei eine mit entsprechenden Fluorochromen beschichtete Glüh- oder Gasentladungslampe (niedrigdruck- oder hochdruckentladend), Licht-emittierende Diode (LED), organische Licht-emittierende Diode (OLED), LASER oder jede andere elektromagnetische Strahlungsquelle sein, die in der Lage ist, aus entsprechenden chemischen Vorstufen bzw. Substraten NO zu generieren.

Für eine optimale Spaltung der in der Badelösung vorhandenen photolabilen NO-Vorstufen kann die Lichtquelle elektromagnetische Strahlung mit Wellenlängen von 100 bis 2000 nm emittieren oder elektromagnetische Strahlung jeder anderen Wellenlänge emittieren, die allein oder mit Unterstützung chemischer, physikalischer oder biologischer Verfahren eine Spaltung von Stickstoffmonoxid-Vorstufen und dadurch eine Bildung von Stickstoffmonoxid induzieren kann.

Vorzugsweise sollte bei einer photolytischen Spaltung daher die Vorrichtung in dem Bestrahlungsbereich aus einem Material aufgebaut sein, das die Eigenschaften des für eine optimale Freisetzung von Stickstoffmonoxid notwendige Energie einer elektromagnetischen Strahlungsquelle nicht beeinflusst oder aufgrund seiner Eigenschaften, die für eine lichtinduzierte Freisetzung von Stickstoffmonoxid notwendigen Lichteigenschaften erst schafft oder optimiert oder im Falle der pH-abhängigen NO-Generierung den pH-induzierten Nitritzerfall fördert und optimiert.

Das zur Bestrahlung des photolabilen NO-Donors eingesetzte Licht liegt in einem Wellenlängenbereich, der von dem jeweiligen NO-Donor abhängig ist. So werden Nitrite zur Photolyse mit UV-Licht in einem Wellenlängenbereich zwischen 320 und 400 nm, bevorzugt zwischen 340 und 380 nm und besonders bevorzugt bei 365 nm bestrahlt. Bei S-Nitroso-Verbindungen ist eine Bestrahlung im $UV_A$-Bereich bevorzugt (d.h. bei Wellenlängen zwischen 315 und 380 nm) aber auch Licht mit einer Wellenlänge von bis zu 1000 nm kann hierzu einer signifikanten Zerfallsrate führen.

Beachtlicherweise ist die optimale Wellenlänge für die Photolyse stark von Metallkationen abhängig. Insbesondere in der Anwesenheit von Ionen von Übergangsmetallen, so z. B. $Cu^{2+}$, können wässrige Nitritlösungen bei wesentlich längeren Wellenlängen Licht absorbieren als es bei "reinen" Nitritlösungen der Fall ist und somit das Nitrition auch durch Licht in Wellenlängen von 400 - 450 nm und noch anderen Wellenlängen $\geq$ 450 nm unter NO-Freisetzung gespalten werden. Auch bei S- und N-nitrosierten chemischen Verbindungen kann aufgrund der relativ schwachen Bindungsenergie zwischen NO und dem Restmolekül ebenfalls durch elektromagnetische Strahlung $\geq$400 nm diese Verbindungen unter NO-Freisetzung photolytisch gespaltet werden.

## Therapeutische oder kosmetische Verwendung

In einem besonderen Aspekt stellt die Erfindung somit eine Badevorrichtung bereit, die zur Verwendung bei der Behandlung oder Prävention von Erkrankungen geeignet ist, wobei mindestens eine Körperextremität des Patienten der NO-haltigen Badelösung ausgesetzt wird.

Erfindungsgemäß wird bei der Badevorrichtung die erkrankte Körperextremität durch Besprühen, Begießen oder Übergießen mit der NO-haltigen Flüssigkeit behandelt.

Die erfindungsgemäße Badevorrichtung kann hierbei insbesondere zur Anregung des Stoffwechsels von Geweben durch äußerliche Anwendung, im Bereich der Dermatologie zur Behandlung von chirurgischen oder unfallbedingten Wunden, chronischen, nicht- bzw. schlechtheilenden und/oder bakteriell bzw. Pilz-befallenen Wunden sowie zur Behandlung von dermatologischen Erkrankungen aus dem Formenkreis der entzündlichen, immunologisch-gesteuerten bzw. Autoimmunerkrankungen verwendet werden.

In einer bevorzugten Ausführungsform ist die mit der erfindungsgemäßen Vorrichtung behandelte Erkrankung ausgewählt aus der Gruppe enthaltend neuropathische Schmerzen, Krampfadern, Ischämien und thrombopathische Erkrankungen, Allergien, Hautinfektionen, Hautentzündungen, atopischen Dermatitis insbesondere Neurodermitis, Dermatomyositis und Pemphigus vulgaris; Wunddefekte, wie der chronischen diabetisch-neuropathischen Ulcus, Ulcus cruris, Dekubituswunden; primär heilende Wunden, sekundär heilende infizierte Wunden, Brandwunden, Hidradenitis supparativa (Akne inversa), Warzen, Windelausschlag, Rasurbrand, Komplikationen bei Hauttransplantationen, erektile Dysfunktion, Angina pectoris, Herzinsuffizienz, Linksherzinsuffizienz, Koronare Herzkrankheit, Pektanginöse Symptome nach Myokardinfarkt, Analfissur, Krämpfe der glatten Muskulatur des Oesophagus, Menstruationsbeschwerden, Reynaud-Syndrom, Buerger-Syndrom, periphere arterielle Erkrankung (PAD), periphere arterielle Verschlusskrankheit (pAVK), entzündliche und Autoimmunerkrankungen der Haut (Psoriasis, Dermatiden, Neurodermitis), Pilzerkrankungen der Haut, bakterielle, mikotische und parasitäre Erkrankungen der Haut (z.B. Leishmaniose), Tinea cruris und Tinea inguinalis.

In einer Ausführungsform können mit der erfindungsgemäßen Badevorrichtung lokale Durchblutungserkrankungen beim Tier, wie beispielsweise die Hufrehe beim Pferd behandelt werden und darüber hinaus generell veterinärmedizinische Erkrankungen, die den hier aufgelisteten Humanerkrankungen entsprechen oder nahekommen.

Die erfindungsgemäße Badevorrichtung kann auch zur Behandlung einer Muskeldystrophie (MD) eingesetzt werden. Behandelbare MD-Formen umfassen hier: MD-Duchenne, MD-Becker-Kiener, Emery-Dreifuss_MD-Typ 1, Skapulopereonale MD, reducing body myopathy (RBM), Gliedergürteldystrophien, kongenitale Muskeldystrophien, distale

Muskeldystrophien, "Vocal cord and pharyngeal weakness with distal myopathy" (VCPDM), Myofibrilläre Myopathien und Myotone Dystrophien,

Eine mit der erfindungsgemäßen Badevorrichtung behandelbare Entzündung kann eine bakterielle, virale, mykotische oder parasitäre Infektion darstellen. Die bakterielle Infektion kann hierbei beispielsweise durch ein Bakterium verursacht werden, das ausgewählt ist aus der Gruppe enthaltend S. aureus, B. circulans, B. cereus, E. coli, P. vulgaris, P. acnes, S. pyogenes, S. enterica, V. anguillarum, K. pneumoniae, P. piscicida, P. aeruginosa, A. tumefaciens, M tuberculosis, and M ulcerans. Die Pilzinfektion kann durch einen Pilz hervorgerufen werden, der ausgewählt ist aus der Gruppe enthaltend T. equinum, C. Albicans, F. oxysporum, R. solani, B. cinerea, und A. jlavus. Bei der zu behandelnden Pilzinfektion kann die Haut oder ein Nagel gemäß einer Onychomycosis befallen sein. Virale Infektionen können durch eine der folgenden Virenfamilien hervorgerufen werden: Poxviridae, Rotaviren, Papillomaviren, Parvoviren, und Varicella-Viren. Bevorzugt kann die NO-freisetzende Vorrichtung zur Behandlung von Hautinfektionen eingesetzt werden, bei denen der Virus Molluscum contagiosum involviert ist. Die parasitäre Infektion kann beispielsweise durch einen Parasit der folgenden Gattungen entstehen: Plasmodium, Leishmania, Schistosoma, Austrobilharzia, Heterobilharzia, Ornithobilharzia oder Cryptosporidium. Hervorzuheben ist hier der Erreger Plasmodium falciparum.

Mit dem erfindungsgemäßen Verfahren hergestellte Lösungen können bevorzugt in Form eines Inhalationssprays zur Behandlung obstruktiver Lungenerkrankungen verwendet werden. Weiterhin können sie zur Induktion einer lokalen Vasodilatation von verengten oder verschlossenen Blutgefäßen eingesetzt werden. Hierbei ist es bevorzugt, die Lösung direkt in das Herz zu applizieren, beispielsweise durch eine endoskopische Maßnahme.

In einer Ausführungsform kann die erfindungsgemäße Badevorrichtung zur Behandlung der anfallsartigen bei der Sichelzellanämie auftretenden Durchblutungsstörungen (Sichelzellkrisen) verwendet werden. Für den in solchen Fällen eingesetzten Wirkstoff Hydroxyharnstoff wird vermutet, dass er bei den Erythrozyten die Ausbildung der deoxygenierten T-Variante hemmt, und so die Umwandlung in den Sichelzellphänotyp verhindert. Durch Anbindung des freigesetzten NOs an das Hämoglobin entsteht hingegen die nicht-sichelzellbildende R-Variante was mit einer Verbesserung der Durchblutung und sogar einer Unterbindung von Sichelzellkrisen einhergehen kann.

In einer weiteren Ausführungsform kann die erfindungsgemäße Badevorrichtung zur Behandlung von Haarausfall und hierbei insbesondere der androgenetischen Alopezie eingesetzt werden. Die Behandlung schließt hier sowohl eine Verlangsamung oder einen Stopp des Haarausfalls mit ein und sogar das Neuwachstum von Haaren. Weitere Formen des Haarausfalls, die erfindungsgemäß behandelt werden können, umfassen Alopecia praematura, Alopecia areata, Alopecia areata atrophicans, Alopecia totalis, Alopecia universalis, diffuse Alopezie, Alopecia actinica, Alopecia mechanis wie Alopecia liminaris, Alopecia marginalis frontalis traumatica, Alopecia seborrhoica, Alopecia muciosa und Alopecia parvimaculata. Analog zur Wirkungsweise des Medikaments Minoxidil sollte das NO hierbei durch erhöhte Durchblutung der Kopfhaut eine verstärkte Versorgung der Haarfollikel mit Blut, Sauerstoff und Nährstoffen mit sich bringen.

Erfindungsgemäß kann die Badevorrichtung beispielsweise wie folgt angewendet werden:

1.) Auf offenen Wunden, da sich überraschenderweise herausgestellt hat, dass die erfindungsgemäße Anwendung nicht hautirritierend wirken;

2.) Für die MRSA Prophylaxe in Risikopatienten; oder

3.) Als synergistische Anwendung mit konventionellen Antibiotika, da sich überraschend herausgestellt hat, dass in Folge einer NO-Einwirkung, die konventionellen Antibiotika die verbleibende Entzündung effektiv zu bekämpfen vermag.

In einer bevorzugten Ausführungsform wird die erfindungsgemäße Badevorrichtung zur Behandlung chronischer Wunden der unteren Extremitäten von Diabetikern verwendet. Hierbei kann zudem durch die Behandlung im Sinne einer Prophylaxe das Entstehungsrisiko chronischer Wunden sowie die Anzahl medizinischer Amputationen verringert werden. Dadurch gehen die Reduktion der neuropathischen Beinschmerzen und die Herstellung eines verbesserten Wundmilieus mit einer spürbar verbesserten Lebensqualität der Patienten einher. Darüber hinaus ist durch eine Verkürzung der Wundversorgung eine signifikante Minderung der Behandlungskosten zu erwarten.

Zudem könnte es möglich sein, durch Behandlung größerer Körperareale auch systemische Erkrankungen, wie z. B. den erhöhten Blutdruck (Hypertonie) und verwandte hämodynamische Erkrankungen zu adressieren.

In einer Ausführungsform der Erfindung wird die erfindungsgemäße Badevorrichtung zur Behandlung schlechtheilender Wunden eingesetzt. Eine gestörte arterielle Durchblutung und/oder venöse Rückflussstörungen sind maßgebliche Ursachen in der Entstehung sowie Chronizität von Wunden der unteren Extremitäten. Eine NO-bedingte arterielle Vasodilatation verbessert die Durchblutung des betroffenen Gewebes und durch die antithrombogene Wirkung des NO wird ein venöser Rückfluss des Blutes wesentlich gefördert bzw. erleichtert. Die NO-abhängige Verbesserung beider hämodynamischer Parameter stellt den entscheidenden therapierelevanten Aspekt einer lokalen Wirkung dar, die das Risiko der Entstehung von Wunden signifikant mindert bzw. deren Heilung wesentlich beschleunigt. Das mittels der erfindungsgemäßen Vorrichtung dem zu behandelnden Körperteil zugeführte NO kann daher erfolgreich zur Therapie

schlechtheilender Wunden angewendet werden.

In einer besonderen Ausführungsform wird die erfindungsgemäße Badevorrichtung zur Behandlung des diabetischen Schmerz der unteren Extremitäten, also von Fuß und/oder Bein, eingesetzt. Der diabetische Schmerz ist ein sehr häufiges Ereignis im Verlauf einer Diabeteserkrankung. Der diabetischer Fuß-/Beinschmerz ist ein Ergebnis langandauernder erhöhter Blutglukosekonzentrationen, die die Grundursache für die während einer Diabeteserkrankung beobachteten Nerven- sowie Gefäßschädigung ist. Eine NO-bedingte arterielle Vasodilatation verbessert die Durchblutung des betroffenen Gewebes und hilft die Schmerzweiterleitung im Sinne einer Schmerzminderung zu beeinflussen. Das mit der erfindungsgemäße Badevorrichtung von außen dem Fuß und/oder Bein zugeführte NO kann daher erfolgreich zur Therapie des diabetischen Fuß-/Beinschmerzes angewendet werden.

In einer speziellen Ausführungsform der Erfindung wird die erfindungsgemäße Badevorrichtung zur Behandlung von Patienten mit (Haut)transplantaten und hierbei insbesondere zur Behandlung von schlecht perfundierten Lappenplastiken eingesetzt. Die beiden vorab genannten hämodynamischen Größen, die arterielle Durchblutung sowie der venöse Rückfluss, stellen auch essenzielle Parameter des Therapieerfolgs chirurgischer Lappenplastiken dar. Als Lappenplastiken werden operative plastisch-chirurgische Techniken bezeichnet, die Haut und/oder Gewebe von einer (entbehrlichen) Stelle des gleichen Individuums an eine neue gewünschte Stelle bringen. In der Regel handelt es sich um reine Hautlappen, es kann aber jedes Gewebe mit oder ohne Haut sowohl gestielt (also mit seinen zugehörigen blutversorgenden Gefäßen und Nerven) als auch frei (d. h. mit Anschluss der Blutgefäße an die Blutversorgung der neuen Umgebung) verpflanzt werden. Die funktionelle Akzeptanz des verpflanzten Gewebes ist dabei ausschließlich von der arteriellen Blutversorgung sowie einem geregelten venösem Abfluss abhängig. Eine NO-bedingte arterielle Vasodilatation verbessert die Durchblutung und somit die notwendige Versorgung der Lappenplastik und durch die antithrombogene Wirkung des NO wird ein venöser Abfluss bzw. Rückfluss des Blutes gefördert und erleichtert. Von außen eingesetzte NO-Präparate können daher den Erfolg einer auf Lappenplastik basierten Therapieoption sichern bzw. fördern.

In einer weiteren Ausführungsform stellt die Erfindung auch ein kosmetisches Verfahren bereit, bei dem das durch die erfindungsgemäße Badevorrichtung hergestellte NO auf die Haut des Menschen einwirkt.

## DEFINITIONEN

Erfindungsgemäß ist unter dem Begriff der "Behandlung" jegliche Anwendung der erfindungsgemäßen Vorrichtung am Individuum zu verstehen, die dazu dient, die Erkrankung symptomatisch oder kausal zu lindern oder sogar gänzlich zu unterdrücken oder das Voranschreiten der Erkrankung aufzuhalten, zu verzögern oder hinauszuschieben.

Als Wirkstoff wird im Rahmen der Erfindung eine pharmakologisch aktive Substanz verstanden - im Gegensatz zu den Hilfsstoffen. Der Wirkstoff ist also derjenige Bestandteil der Badelösung, der eventuell im Zusammenspiel mit den Hilfsstoffen, für die Wirksamkeit der Badelösung verantwortlich ist.

Im Kontext der vorliegenden Erfindung wird unter der "Prävention" die Vermeidung des Auftretens von Erkrankungen, und insbesondere von vaskulären oder Stoffwechsel-Erkrankungen verstanden und damit die Verringerung ihrer Verbreitung und die Verminderung ihrer Auswirkungen auf Morbidität und Mortalität der Bevölkerung. Die zentrale Strategie ist, die Auslösefaktoren von Krankheiten zurückzudrängen oder ganz auszuschalten.

Die Prävention umfasst hierbei sowohl primordiale Prävention, die Primärprävention, die Sekundärprävention, die Tertiärprävention und auch die Quartärprävention.

Primärprävention setzt vor Eintreten der Krankheit ein und zielt darauf ab, ein Neuauftreten einer Erkrankung zu verhindern. Die Primärprävention richtet sich an Risikogruppen, Gesunde und Personen ohne Krankheitssymptome.

Von der Primärprävention kann noch die primordiale Prävention abgegrenzt werden, die noch früher einsetzt. Bei ihr geht es darum, bereits dem Auftreten von Risikofaktoren vorzubeugen.

Sekundärprävention setzt beim Frühstadium einer Krankheit an. Sie dient der Früherkennung von Krankheiten und der Eindämmung ihres Fortschreitens (Progredienz) oder der Chronifizierung der Erkrankung. Oft ohne eine für die Betroffenen wahrnehmbare Krankheitssymptomatik hat der pathogenetische Prozess hier bereits seinen Anfang genommen. Zielgruppe sind Personen, die zwar als Gesunde oder Symptomlose an der Präventionsmaßnahme teilnehmen, durch die diagnostische Maßnahme aber zu Patienten werden.

Tertiärprävention findet nach einer Akutbehandlung oder der Manifestation einer Erkrankung statt. Mit ihr sollen Folgeschäden und Rückfälle verhindert werden. Sie richtet sich an Patienten mit chronischen Beeinträchtigungen und an Rehabilitanden. Ein Beispiel ist hier die Verhinderung von Rezidiven bei Tumorerkrankungen.

Weiterhin gibt es noch die Quartäre Prävention, die die Verhinderung unnötiger Medizin oder Verhinderung von Überdosierungen zum Ziel hat und das Prinzip des «primum non nocere» als einen Grundpfeiler aller Medizin berücksichtigt.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

Im Einklang mit der vorangehenden Beschreibung werden die folgenden Ausführungsformen offenbart, die alleine oder in jeglicher Kombination mit dem vorgenannten Ausführungsformen ebenfalls Gegenstand der Erfindung sind.

Die Ausführungsform 1 betrifft eine medizinische Badevorrichtung zur Behandlung von Körperextremitäten mit einer wirkstoffhaltigen Badelösung umfassend:

(a) Eine Behandlungskammer zur Aufnahme einer oder mehrerer Körperextremitäten;
(b) Ein Reaktionsgefäß zur Herstellung der wirkstoffhaltigen Badelösung;
(c) Ein System zum Pumpen und/oder Umwälzen der wirkstoffhaltigen Badelösung;
(d) Eine Duschvorrichtung; und
(e) Ein Gefäß zur Aufnahme der verbrauchten Badelösung;

wobei die in dem Reaktionsgefäß hergestellte wirkstoffhaltige Badelösung durch das Umwälzungs-/Pumpsystem zu der Duschvorrichtung transportiert wird.

Ausführungsform 2: Medizinische Badevorrichtung gemäß Ausführungsform 1, dadurch gekennzeichnet, dass das Gefäß zur Aufnahme der verbrauchten Badelösung dem Reaktionsgefäß entspricht.

Ausführungsform 3: Medizinische Badevorrichtung gemäß Ausführungsform 1 oder 2, dadurch gekennzeichnet, dass das Reaktionsgefäß und die Behandlungskammer als eigenständige Gefäße über eine Flüssigkeitsleitung miteinander verbunden ist.

Ausführungsform 4: Medizinische Badevorrichtung gemäß einer der Ausführungsformen 1 bis 3, dadurch gekennzeichnet, dass das Reaktionsgefäß als geschlossener Behälter ausgebildet ist, der jeweils wenigstens einen Zulauf und einen Ablauf für die Badelösung aufweist.

Ausführungsform 5: Medizinische Badevorrichtung gemäß einer der Ausführungsformen 1 bis 4, dadurch gekennzeichnet, dass das Reaktionsgefäß zusätzlich eine oder mehrere Lichtquellen umfasst, die bevorzugt UV-Lichtquellen sind.

Ausführungsform 6: Medizinische Badevorrichtung gemäß einer der Ausführungsformen 1 bis 5, dadurch gekennzeichnet, dass die Duschvorrichtung ein tragbarer Duschkopf ist.

Ausführungsform 7: Medizinische Badevorrichtung gemäß einer der Ausführungsformen 1 bis 6, dadurch gekennzeichnet, dass die Behandlungskammer zusätzlich eine Auflage zum Aufsetzen der mindestens einen Körperextremität umfasst, wobei die Auflage mindestens eine Öffnung zum Abfließen der Badelösung enthält.

Ausführungsform 8: Medizinische Badevorrichtung gemäß Ausführungsform 7, dadurch gekennzeichnet, dass in der Behandlungskammer unterhalb der Auflage eine oder mehrere Trennabschnitte mit mindestens einer Öffnung zum Abfließen der Badelösung enthalten sind, wobei deren mindestens eine Öffnung bevorzugt kleiner ist als die die mindestens eine Öffnung der Auflage.

Ausführungsvorrichtung 9: Medizinische Badevorrichtung gemäß einer der Ausführungsformen 1 bis 8, dadurch gekennzeichnet, dass das Gefäß zur Aufnahme der verbrauchten Badelösung unterhalb der Behandlungskammer angebracht ist und mit der Behandlungskammer flüssigkeitsführend verbunden ist, wobei die Behandlungskammer bevorzugt von dem unteren Gefäß abnehmbar ist.

Ausführungsform 10: Medizinische Badevorrichtung gemäß einer der Ausführungsformen 1 bis 9, dadurch gekennzeichnet, dass die Badevorrichtung bodenseitig Rollen oder Räder aufweist.

Ausführungsform 11: Medizinische Badevorrichtung zur Behandlung von Körperextremitäten mit einer wirkstoffhaltigen Badelösung umfassend:

(a) Einen an einen Wasserhahn direkt oder über eine Leitung anschließbares Rektionsgefäß zur Erzeugung einer wirkstoffhaltigen Badelösung; und
(b) einen tragbaren Duschkopf zur Abgabe der wirkstoffhaltigen Badelösung;

wobei der Duschkopf über eine Leitung mit dem Reaktionsgefäß verbunden ist oder das Reaktionsgefäß umfasst.

Ausführungsform 12: Medizinische Badevorrichtung gemäß einer der Ausführungsformen 1 bis 11, dadurch gekennzeichnet, dass die wirkstoffhaltige Badelösung eine Stickstoffmonoxid (NO)-haltige Badelösung ist.

Ausführungsform 13: Medizinische Badevorrichtung gemäß Ausführungsform 12, dadurch gekennzeichnet, dass in dem Reaktionsgefäß eine NO-haltigen Badelösung gemäß einem Verfahren hergestellt wird, dass die folgenden Schritte umfasst:

(a) Bereitstellen einer Badelösung umfassend mindestens einen pH-labilen NO-Donor;
(b) Einstellen des pH-Werts der Badelösung auf einen pH-Wert, der die Zersetzung des mindestens einen pH-labilen NO-Donors unter Bildung von NO induziert;
(c) Aufrechterhalten eines die NO-Bildung induzierenden pH-Werts für eine Zeitdauer, die die Bildung einer physiologisch relevanten Menge an NO erlaubt;
(d) Erhöhen des pH-Werts der Badelösung;
(e) Optionale Zugabe eines weiteren mindestens einen Antioxidans;

wobei die Badelösung in Schritt (a) zusätzlich mindestens ein Antioxidans enthält oder das mindestens eine Antioxidans in Schritt (b) hinzugegeben wird.

Ausführungsform 14: Verwendung einer medizinischen Badevorrichtung gemäß einer der Ausführungsformen 1 bis 12 zur Herstellung einer NO-haltigen Badelösung, wobei das Verfahren zur Herstellung der NO-haltigen Badelösung die folgenden Schritte umfasst:

(a) Bereitstellen einer Badelösung umfassend mindestens einen pH-labilen NO-Donor;
(b) Einstellen des pH-Werts der Badelösung auf einen pH-Wert, der die Zersetzung des mindestens einen pH-labilen NO-Donors unter Bildung von NO induziert;
(c) Aufrechterhalten eines die NO-Bildung induzierenden pH-Werts für eine Zeitdauer, die die Bildung einer physiologisch relevanten Menge an NO erlaubt;
(d) Erhöhen des pH-Werts der Badelösung;

Ausführungsform 15: Medizinische Badevorrichtung gemäß Ausführungsform 12 oder 13, dadurch gekennzeichnet, dass nach Schritt (d) oder (e) die Badelösung zur photolytischen Zersetzung des NO-Donors unter Bildung von NO mit Licht bestrahlt wird.

Ausführungsform 16: Medizinische Badevorrichtung gemäß Ausführungsform 13 bis 15 zur Verwendung bei der Behandlung oder Prävention von Erkrankungen, dadurch gekennzeichnet, dass die mindestens eine Körperextremität des Patienten dem aus der Badevorrichtung freigesetzten NO ausgesetzt wird.

Ausführungsform 17: Medizinische Badevorrichtung gemäß Ausführungsform 16, dadurch gekennzeichnet, dass die Erkrankung ausgewählt ist aus der Gruppe enthaltend neuropathische Schmerzen, Krampfadern, Ischämien und thrombopathische Erkrankungen, Allergien, Hautinfektionen, Hautentzündungen, atopischen Dermatitis insbesondere Neurodermitis, Dermatomyositis und Pemphigus vulgaris; Wunddefekte, wie der chronischen diabetisch-neuropathischen Ulcus, Ulcus cruris, Dekubituswunden; primär heilende Wunden, sekundär heilende infizierter Wunden, Komplikationen bei Hauttransplantationen, erektile Dysfunktion, Hidradenitis supparativa (Akne inversa), Warzen, Windelausschlag, Rasurbrand, Reynaud-Syndrom, Buerger-Syndrom, periphere arterielle Erkrankung (PAD), periphere arterielle Verschlusskrankheit (pAVK), entzündliche und Autoimmunerkrankungen der Haut (Psoriasis, Dermatiden, Neurodermitis), Pilzerkrankungen der Haut, Bakterielle, mikotische und Parasitäre Erkrankungen der Haut (z.B. Leishmaniose), Tinea cruris, Tinea, inguinalis, Muskeldystrophien, Sichelzellanämie und Alopezie.

Ausführungsform 18: Medizinische Badevorrichtung gemäß Ausführungsform 17, dadurch gekennzeichnet, dass sie zur Behandlung chronischer Wunden der unteren Extremitäten von Diabetikern verwendet wird.

Ausführungsform 19: Kosmetisches Verfahren umfassend die Einwirkung von NO auf die Haut eines Menschen, dadurch gekennzeichnet, dass eine medizinische Badevorrichtung gemäß einer der Ausführungsformen 1 bis 12 verwendet wird.

## BEISPIELE

### Beispiel 1. Badevorrichtung mit einem einstufigen pH-induzierten NO-Herstellungsverfahren

#### 1.1 Material:

- Eco physics CLD 822: Quantifizierung von NO
- Reaktionskammer: Quarzglas, ca. 100x100x10mm (ca. 100ml Volumen)
- Pufferlösung: 150 mM Essigsäure, 150 mM NaOH in Aqua-dest
- Base: 1M NaOH
- Natrium L-Ascorbat
- 1M NaNO2

#### 1.2 Versuchsführung

0,56 g Natrium-L-ascorbat wurden in 98,6 ml Pufferlösung gelöst, in die Reaktionskammer überführt und 1,4 ml $NaNO_2$ (1M) zugegeben. Die Natriumnitritkonzentration betrug demnach 14 mM, die Ascorbatkonzentration 28,3 mM. Für die Endlösung wurde ein pH-Wert von 5,0 gemessen.

Über einen Zeitraum von 60 min wurde in Abständen von jeweils 2-3 Minuten eine 200 µl Probe entnommen und der NO Gehalt mit Hilfe des CLD Systems quantifiziert.

#### 1.3 Ergebnisse

Die Ergebnisse der NO-Messungen in Abhängigkeit von der Reaktionszeit sind in Figur 1 dargestellt. Es ist ein kontinuierlicher Anstieg der NO-Konzentration zu beobachten, wobei nach 60 Minuten ein Wert erreicht wird, der einer Konzentration von 1,11 mM in der Flüssigkeit entspricht.

### Beispiel 2. Badevorrichtung mit zweistufigem, pH-induzierten NO-Herstellungsverfahren

Ziel dieses Versuchs war das Erreichen einer therapeutisch relevanten Endkonzentration über einen langen Zeitraum durch aktive Änderung des pH-Wertes.

#### 2.1 Material:

Es wurde das gleiche Material wie in Beispiel 1 verwendet.

#### 2.2 Versuchsführung

Es wurde zunächst analog zu Versuch 1 0,56 g Natrium-L-ascorbat in 98,6 ml Pufferlösung gelöst, diese in die Reaktionskammer überführt und 1,4 ml $NaNO_2$ (1M) zugegeben. Die Natriumnitritkonzentration betrug demnach 14 mM, die Ascorbatkonzentration 28,3 mM. Für die Endlösung wurde ein pH-Wert von 5,0 gemessen.

Über einen Zeitraum von insg. 45 min wurde in Abständen von jeweils 2-3 Minuten eine 200 µl Probe entnommen und der NO Gehalt mit Hilfe des CLD Systems quantifiziert.

#### 2.3 Ergebnisse

Es ist zunächst ein kontinuierlicher Anstieg der NO-Konzentration zu beobachten, wobei im Zeitraum zwischen 10-15 min schrittweise insg. 1,5 ml NaOH (1M) zur Reaktionskammer zugegeben wurden, was eine finale pH-Wert Änderung auf pH 5,6 zur Folge hatte. Die NO-Konzentration sinkt in Folge zunächst und pendelt sich ab t = 20 min bei einem Wert von 250 +/- 50 µM ein.

## FIGURENLEGENDEN

Die Erfindung wird nachfolgend anhand der Figuren näher erläutert ohne die Erfindung auf dieses zu beschränken. Es zeigen:

Fig. 1: die Generierung von NO mittels Natriumnitrit als NO-Donor in einem Acetat-Puffer unter Anwesenheit von Ascorbat als Antioxidans bei pH 5.0 über einen Zeitraum von einer Stunde (siehe Beispiel 1).

Fig. 2: die Generierung von NO gemäß der Erfindung mittels Natriumnitrit als NO-Donor in einem Acetat-Puffer unter Anwesenheit von Ascorbat als Antioxidans mit einer ersten Phase von t=0 bis t= 600 sec bei pH 5.0, einer pH-Erhöhung von t=600 bis t=900 auf einen pH-Wert von 5.6 und einer anschließenden Phase der NO-Generierung bei diesem pH-Wert von 5.6 (siehe Beispiel 2).

Fig. 3: eine erfindungsgemäße medizinische Badevorrichtung mit einer Schlauchleitung (5), die an einen Wasserhahn (1) angeschlossen ist und bei Durchleitung eines Reaktionsgefäßes (4) mit NO angereichert wird, wobei die NO-haltige Badelösung danach einem mit einem Schalter (6) versehenen Duschkopf (7) zugeführt wird und von diesem freigesetzt wird (8). Dem Rektionsgefäß sind ein Filter (2) und ein Druckregulierer (3) vorgeschaltet.

Fig. 4: eine als Fußbad angewendete erfindungsgemäße medizinische Badevorrichtung mit einer Behandlungskammer (8), einer Fuß-Auflage (10), zusätzlichen, als Filter fungierenden Trennabschnitten (11), wobei der unterhalb der Auflage gelegene Bereich der Behandlungskammer als Gefäß zur Aufnahme der verbrauchten Badelösung (13) dient. In diesem Bereich ist eine Pumpe (14) installiert, die die verbrauchte Badelösung über eine Schlauchleitung (5) in das separate Reaktionsgefäß (4) pumpt, wo es unter optionalem Einsatz von UV-Lichtquellen (11) wieder regeneriert wird. Die regenerierte, d.h. ausreichend wirkstoffhaltige, Badelösung wird über eine im Reaktionsgefäß angeordnete Pumpe (14) und eine Schlauchleitung (5) dem mit einem Schalter (6) versehenen Duschkopf (7) zugeführt. Sowohl die Behandlungskammer als auch das Reaktionsgefäß sind mit Rollen (15) versehen.

Fig. 5: eine als Fußbad angewendete medizinische Badevorrichtung mit einer Behandlungskammer (8), einer Fuß-Auflage (10), wobei unterhalb Behandlungskammer ein abnehmbares Gefäß zur Aufnahme der verbrauchten Badelösung (13) angeordnet ist. Die NO-haltige Badelösung wird in einem separaten Reaktionsgefäß (4) unter optionalem Einsatz von UV-Lichtquellen (11) hergestellt. Die so hergestellte NO-haltige, Badelösung wird über eine im Reaktionsgefäß angeordnete Pumpe (14) und eine Schlauchleitung (5) dem mit einem Schalter (6) versehenen Duschkopf (7) zugeführt. Sowohl die Behandlungskammer als auch das Reaktionsgefäß sind mit Rollen (15) versehen.

**BEZUGSZEICHEN**

| | |
|---|---|
| 1 | Wasserhahn |
| 2 | Filter |
| 3 | Druckregulierer |
| 4 | Reaktionsgefäß |
| 5 | Schlauchleitung |
| 6 | Schalter |
| 7 | Duschkopf |
| 8 | wirkstoffhaltige Badelösung (mit NO als bevorzugtem Wirkstoff) |
| 9 | Behandlungskammer |
| 10 | Auflage für den Fuß |
| 11 | Trennabschnitte |
| 12 | Wasserpumpe |
| 13 | Gefäß zur Aufnahme der verbrauchten Badelösung |
| 14 | UV-Lichtquellen |
| 15 | Rollen |
| 16 | verbrauchte Badelösung |

**Patentansprüche**

**1.** Medizinische Badevorrichtung zur Behandlung von Körperextremitäten mit einer wirkstoffhaltigen flüssigen Badelösung (8) umfassend:

(a) Eine nach oben offene Behandlungskammer (9) zur Aufnahme einer oder mehrerer Körperextremitäten;
(b) Ein Reaktionsgefäß (4) zur Herstellung der wirkstoffhaltigen Badelösung;
(c) Ein System (12) zum Pumpen und/oder Umwälzen der wirkstoffhaltigen Badelösung (8);
(d) Eine Duschvorrichtung; und

(e) Ein Gefäß oder eine Vorrichtung zur Aufnahme der verbrauchten Badelösung (13), die mit der Behandlungskammer (9) flüssigkeitsführend und abnehmbar verbunden ist;

wobei die in dem Reaktionsgefäß hergestellte wirkstoffhaltige Badelösung (8) über eine Schlauchleitung (5) durch das Umwälzungs-/Pumpsystem (12) zu der Duschvorrichtung transportiert wird, **dadurch gekennzeichnet, dass** das Reaktionsgefäß (4) als geschlossener Behälter ausgebildet ist, der jeweils wenigstens einen Zulauf und einen Ablauf für die Badelösung aufweist und zusätzlich eine oder mehrere UV-Lichtquellen (14) zur Photolyse von NO-Donoren in der Badelösung aufweist; und die Duschvorrichtung ein tragbarer Duschkopf (7) ist, der separat von der Behandlungskammer (9) ausgestaltet ist.

2. Medizinische Badevorrichtung gemäß Anspruch 1, **dadurch gekennzeichnet, dass** das Gefäß zur Aufnahme der verbrauchten Badelösung (13) dem Reaktionsgefäß (4) entspricht.

3. Medizinische Badevorrichtung gemäß Anspruch 1 oder 2, **dadurch gekennzeichnet, dass** das Reaktionsgefäß (4) und die Behandlungskammer (9) als eigenständige Gefäße über eine Flüssigkeitsleitung miteinander verbunden ist.

4. Medizinische Badevorrichtung gemäß einem der vorangehenden Ansprüche, **dadurch gekennzeichnet, dass** der tragbare Duschkopf (7) mit einem Schalter (6) ausgestattet ist, der die Wasserzufuhr regelt.

5. Medizinische Badevorrichtung gemäß einem der vorangehenden Ansprüche, **dadurch gekennzeichnet, dass** die Behandlungskammer (9) zusätzlich eine Auflage (10) zum Aufsetzen der mindestens einen Körperextremität umfasst, wobei die Auflage (10) mindestens eine Öffnung zum Abfließen der Badelösung enthält.

6. Medizinische Badevorrichtung gemäß Anspruch 5, **dadurch gekennzeichnet, dass** in der Behandlungskammer (9) unterhalb der Auflage (10) eine oder mehrere Trennabschnitte (11) mit mindestens einer Öffnung zum Abfließen der Badelösung enthalten sind, wobei deren mindestens eine Öffnung bevorzugt kleiner ist als die die mindestens eine Öffnung der Auflage (10).

7. Medizinische Badevorrichtung gemäß einem der vorangehenden Ansprüche, **dadurch gekennzeichnet, dass** das Gefäß zur Aufnahme der verbrauchten Badelösung (13) unterhalb, neben oder oberhalb der Behandlungskammer (9) angebracht ist.

8. Medizinische Badevorrichtung gemäß einem der vorangehenden Ansprüche, **dadurch gekennzeichnet, dass** die Badelösung (8) mittels Gravitation von der Behandlungskammer (9) in das Gefäß oder die Vorrichtung zur Aufnahme der verbrauchten Badelösung (13) abfließt.

9. Medizinische Badevorrichtung gemäß einem der Ansprüche 1 bis 8, **dadurch gekennzeichnet, dass** die flüssigkeitsführende Verbindung zwischen der Behandlungskammer und dem Gefäß oder der Vorrichtung zu Aufnahme der verbrauchten Badelösung eine Pumpvorrichtung (12) umfasst.

10. Medizinische Badevorrichtung gemäß einem der vorangehenden Ansprüche, **dadurch gekennzeichnet, dass** die flüssigkeitsführende Verbindung zwischen der Behandlungskammer (9) und dem Gefäß oder der Vorrichtung zu Aufnahme der verbrauchten Badelösung (13) eine Filtervorrichtung und/oder eine Absorptionsvorrichtung zur Aufreinigung der verbrauchten Badelösung umfasst.

11. Medizinische Badevorrichtung gemäß einem der vorangehenden Ansprüche, **dadurch gekennzeichnet, dass** das Gefäß oder die Vorrichtung zur Aufnahme der verbrauchten Badelösung (13) superabsorbierendes Material umfasst.

12. Medizinische Badevorrichtung gemäß einem der vorangehenden Ansprüche, **dadurch gekennzeichnet, dass** die Vorrichtung zur Aufnahme der verbrauchten Badelösung (13) eine Flüssigkeitsleitung zur Weiterleitung an eine von der Badevorrichtung getrennte Entsorgungseinheit darstellt.

13. Medizinische Badevorrichtung gemäß einem der vorangehenden Ansprüche, **dadurch gekennzeichnet, dass** die Badevorrichtung bodenseitig Rollen (15) oder Räder aufweist.

14. Medizinische Badevorrichtung gemäß einem der vorangehenden Ansprüche, **dadurch gekennzeichnet, dass** die wirkstoffhaltige Badelösung (8) eine Stickstoffmonoxid (NO)-haltige Badelösung ist.

**15.** Verwendung einer medizinischen Badevorrichtung gemäß einem der Ansprüche 1 bis 14 zur Herstellung einer NO-haltigen Badelösung, wobei das Verfahren zur Herstellung der NO-haltigen Badelösung die folgenden Schritte umfasst:

(a) Bereitstellen einer Badelösung umfassend mindestens einen pH-labilen NO-Donor;
(b) Einstellen des pH-Werts der Badelösung auf einen pH-Wert, der die Zersetzung des mindestens einen pH-labilen NO-Donors unter Bildung von NO induziert;
(c) Aufrechterhalten eines die NO-Bildung induzierenden pH-Werts für eine Zeitdauer, die die Bildung einer physiologisch relevanten Menge an NO erlaubt;
(d) Erhöhen des pH-Werts der Badelösung um mindestens eine pH-Wertstufe.

**16.** Verwendung der medizinischen Badevorrichtung gemäß Anspruch 15, **dadurch gekennzeichnet, dass** nach Schritt (d) oder (e) die Badelösung zur photolytischen Zersetzung des NO-Donors unter Bildung von NO mit Licht bestrahlt wird.

**17.** Medizinische Badevorrichtung gemäß einem der Ansprüche 1 bis 14 zur Verwendung bei der Behandlung oder Prävention von Erkrankungen, **dadurch gekennzeichnet, dass** die mindestens eine Körperextremität des Patienten dem aus der Badevorrichtung freigesetzten NO ausgesetzt wird, wobei die Duschvorrichtung an die jeweilige Anwendung angepasst werden kann.

**18.** Medizinische Badevorrichtung gemäß Anspruch 17, **dadurch gekennzeichnet, dass** die Erkrankung ausgewählt ist aus der Gruppe enthaltend neuropathische Schmerzen, Krampfadern, Ischämien und thrombopathische Erkrankungen, Allergien, Hautinfektionen, Hautentzündungen, atopischen Dermatitis insbesondere Neurodermitis, Dermatomyositis und Pemphigus vulgaris; Wunddefekte, wie der chronischen diabetisch-neuropathischen Ulcus, Ulcus cruris, Dekubituswunden; primär heilende Wunden, sekundär heilende infizierter Wunden, Komplikationen bei Hauttransplantationen, erektile Dysfunktion, Hidradenitis supparativa (Akne inversa), Warzen, Windelausschlag, Rasurbrand, Reynaud-Syndrom, Buerger-Syndrom, periphere arterielle Erkrankung (PAD), periphere arterielle Verschlusskrankheit (pAVK), entzündliche und Autoimmunerkrankungen der Haut (Psoriasis, Dermatiden, Neurodermitis), Pilzerkrankungen der Haut, Bakterielle, mikotische und Parasitäre Erkrankungen der Haut (z.B. Leishmaniose), Tinea cruris, Tinea, inguinalis, Muskeldystrophien, Sichelzellanämie und Alopezie, wobei die Duschvorrichtung an die jeweilige Anwendung angepasst werden kann.

**19.** Medizinische Badevorrichtung gemäß Anspruch 18, **dadurch gekennzeichnet, dass** sie zur Behandlung chronischer Wunden der unteren Extremitäten von Diabetikern verwendet wird, wobei die Duschvorrichtung an die jeweilige Anwendung angepasst werden kann.

**20.** Kosmetisches Verfahren umfassend die Einwirkung von NO auf die Haut eines Menschen, **dadurch gekennzeichnet, dass** eine medizinische Badevorrichtung gemäß einem der Ansprüche 1 bis 14 zur Herstellung einer NO-haltigen Badelösung für die Einwirkung von NO auf die Haut eines Menschen verwendet wird.

## Claims

**1.** Medical bathing device for the treatment of body limbs with an active substance-containing liquid bathing solution (8), comprising:

(a) a treatment chamber (9) that is open at the top for accommodation of one or more body limbs;
(b) a reaction vessel (4) for preparation of the active substance-containing bathing solution;
(c) a system (12) for pumping and/or circulating the active substance-containing bathing solution (8);
(d) a shower device; and
(e) a vessel or a device for accommodation of the spent bathing solution (13), which is connected to the treatment chamber (9) such as to be liquid-conducting and detachable;

whereby the active substance-containing bathing solution (8) prepared in the reaction vessel is transported via a hose line (5) through the circulating/pump system (12) to the shower device, **characterized in that**
the reaction vessel (4) is designed as a closed container that comprises at least one inlet and one outlet each for the bathing solution and in addition comprises one or more UV light sources (14) for photolysis of NO donors in the bathing solution; and the shower device is a portable shower head (7) that is designed to be separate from the

treatment chamber (9).

2. Medical bathing device according to claim 1, **characterized in that** the vessel for accommodation of the spent bathing solution (13) corresponds to the reaction vessel (4).

3. Medical bathing device according to claim 1 or 2, **characterized in that** the reaction vessel (4) and the treatment chamber (9) are connected to each other as independent vessels by means of a liquid line.

4. Medical bathing device according to any one of the preceding claims, **characterized in that** the portable shower head (7) is equipped with a switch (6) that regulates the water feed.

5. Medical bathing device according to any one of the preceding claims, **characterized in that** the treatment chamber (9) comprises, in addition, a support (10) allowing the at least one body limb to be placed on it, whereby the support (10) contains at least one opening for discharge of the bathing solution.

6. Medical bathing device according to claim 5, **characterized in that** the treatment chamber (9) contains, below the support (10), one or more separating sections (11) with at least one opening for discharge of the bathing solution, whereby the at least one opening thereof is preferred to be smaller than the at least one opening of the support (10).

7. Medical bathing device according to any one of the preceding claims, **characterized in that** the vessel for accommodation of the spent bathing solution (13) is attached below, adjacent to or above the treatment chamber (9).

8. Medical bathing device according to any one of the preceding claims, **characterized in that** the bathing solution (8) is discharged from the treatment chamber (9) into the vessel or the device for accommodation of the spent bathing solution (13) by the action of gravity.

9. Medical bathing device according to any one of the claims 1 to 8, **characterized in that** the liquid-conveying connection between the treatment chamber and the vessel or the device for accommodation of the spent bathing solution comprises a pump device (12).

10. Medical bathing device according to any one of the preceding claims, **characterized in that** the liquid-conveying connection between the treatment chamber (9) and the vessel or the device for accommodation of the spent bathing solution (13) comprises a filter device and/or an absorption device for purification of the spent bathing solution.

11. Medical bathing device according to any one of the preceding claims, **characterized in that** the vessel or the device for accommodation of the spent bathing solution (13) comprises super-absorbing material.

12. Medical bathing device according to any one of the preceding claims, **characterized in that** the device for accommodation of the spent bathing solution (13) is a liquid line for conveyance to a disposal unit that is separate from the bathing device.

13. Medical bathing device according to any one of the preceding claims, **characterized in that** the bathing device comprises, on the bottom side, rollers (15) or wheels.

14. Medical bathing device according to any one of the preceding claims, **characterized in that** the active substance-containing bathing solution (8) is a nitrogen monoxide (NO)-containing bathing solution.

15. Use of a medical bathing device according to any one of the claims 1 to 14 for preparation of a NO-containing bathing solution, whereby the method for preparation of the NO-containing bathing solution comprises the following steps of:

(a) providing a bathing solution comprising at least one pH-labile NO donor;
(b) adjusting the pH value of the bathing solution to a pH value that induces the decomposition of the at least one pH-labile NO donor while generating NO;
(c) maintaining a pH value that induces the generation of NO for a period of time that allows for the generation of a physiologically relevant amount of NO;
(d) increasing the pH value of the bathing solution by at least one pH level.

16. Use of the medical bathing device according to claim 15, **characterized in that** the bathing solution is irradiated,

after step (d) or (e), with light for photolytic decomposition of the NO donor while generating NO.

**17.** Medical bathing device according to any one of the claims 1 to 14 for use in the treatment or prevention of diseases, **characterized in that** the at least one body limb of the patient is being exposed to the NO released from the bathing device, whereby the shower device can be adjusted to the respective application.

**18.** Medical bathing device according to claim 17, **characterized in that** the disease is selected from the group containing neuropathic pain, varicose veins, ischemias and thrombopathic diseases, allergies, skin infections, skin inflammations, atopic dermatitis, in particular neurodermatitis, dermatomyositis and pemphigus vulgaris; wound defects, such as chronic diabetic-neuropathic ulcer, ulcer cruris, decubitus wounds; primary healing wounds, secondary healing infected wounds, complications with skin transplants, erectile dysfunction, hidradenitis supparativa (acne inversa), warts, diaper rash, razor burn, Reynaud Syndrome, Buerger Syndrome, peripheral arterial disease (PAD), peripheral arterial occlusive disease (PAOD), inflammatory and autoimmune diseases of the skin (psoriasis, dermatitis, neurodermatitis), fungal skin infections, bacterial, mycotic and parasitic diseases of the skin (e.g., leishmaniasis), tinea cruris, tinea inguinalis, muscular dystrophies, sickle-cell anaemia and alopecia, whereby the shower device can be adjusted to the respective application.

**19.** Medical bathing device according to claim 18, **characterized in that** it is used for the treatment of chronic wounds of the lower limbs of diabetics, whereby the shower device can be adjusted to the respective application.

**20.** Cosmetic method comprising the action of NO on the skin of a human, **characterized in that** a medical bathing device according to any one of the claims 1 to 14 for preparation of a NO-containing bathing solution for the action of NO on the skin of a human is used.

## Revendications

**1.** Dispositif de bain médical pour le traitement d'extrémités corporelles avec une solution de bain liquide contenant des substances actives (8) comprenant :

(a) une chambre de traitement ouverte vers le haut (9) pour la réception d'une ou plusieurs extrémités corporelles ;
(b) un récipient de réaction (4) pour la fabrication de la solution de bain contenant des substances actives ;
(c) un système (12) pour le pompage et/ou la recirculation de la solution de bain contenant des substances actives (8) ;
(d) un dispositif de douche ; et
(e) un récipient ou un dispositif pour la réception de la solution de bain usagée (13) qui est connecté à la chambre de traitement (9) de manière à guider le liquide et amovible ;

dans lequel la solution de bain contenant des substances actives (8) fabriquée dans le récipient de réaction est transportée par le biais d'une tuyauterie souple (5) à travers le système de recirculation/pompage (12) vers le dispositif de douche,
**caractérisé en ce que**
le récipient de réaction (4) est réalisé en tant que récipient fermé qui présente à chaque fois au moins une amenée et une évacuation pour la solution de bain, et présente en outre une ou plusieurs sources de lumière UV (14) pour la photolyse de donneurs de NO dans la solution de bain ; et le dispositif de douche est une tête de douche portable (7) qui est configurée séparément de la chambre de traitement (9).

**2.** Dispositif de bain médical selon la revendication 1, **caractérisé en ce que** le récipient pour la réception de la solution de bain usagée (13) correspond au récipient de réaction (4).

**3.** Dispositif de bain médical selon la revendication 1 ou 2, **caractérisé en ce que** le récipient de réaction (4) et la chambre de traitement (9) sont connectés l'un à l'autre par le biais d'une conduite de liquide en tant que récipients autonomes.

**4.** Dispositif de bain médical selon une des revendications précédentes, **caractérisé en ce que** la tête de douche portable (7) est équipée d'un commutateur (6) qui régule l'arrivée d'eau.

**5.** Dispositif de bain médical selon une des revendications précédentes, **caractérisé en ce que** la chambre de traitement (9) comprend en outre un appui (10) pour la pose de l'au moins une extrémité corporelle, dans lequel l'appui (10) contient au moins une ouverture pour l'écoulement de la solution de bain.

**6.** Dispositif de bain médical selon la revendication 5, **caractérisé en ce qu'**une ou plusieurs sections de séparation (11) avec au moins une ouverture pour l'écoulement de la solution de bain sont contenues dans la chambre de traitement (9) en dessous de l'appui (10), dans lequel leur au moins une ouverture est de préférence plus petite que l'au moins une ouverture de l'appui (10).

**7.** Dispositif de bain médical selon une des revendications précédentes, **caractérisé en ce que** le récipient pour la réception de la solution de bain usagée (13) est monté en dessous, à côté ou au-dessus de la chambre de traitement (9).

**8.** Dispositif de bain médical selon une des revendications précédentes, **caractérisé en ce que** la solution de bain (8) s'écoule au moyen de la gravitation de la chambre de traitement (9) dans le récipient ou le dispositif pour la réception de la solution de bain usagée (13).

**9.** Dispositif de bain médical selon une des revendications 1 à 8, **caractérisé en ce que** la connexion de guidage de liquide entre la chambre de traitement et le récipient ou le dispositif pour la réception de la solution de bain usagée comprend un dispositif de pompe (12).

**10.** Dispositif de bain médical selon une des revendications précédentes, **caractérisé en ce que** la connexion de guidage de liquide entre la chambre de traitement (9) et le récipient ou le dispositif pour la réception de la solution de bain usagée (13) comprend un dispositif de filtre et/ou un dispositif d'absorption pour la filtration de la solution de bain usagée.

**11.** Dispositif de bain médical selon une des revendications précédentes, **caractérisé en ce que** le récipient ou le dispositif pour la réception de la solution de bain usagée (13) comprend un matériau super absorbant.

**12.** Dispositif de bain médical selon une des revendications précédentes, **caractérisé en ce que** le dispositif pour la réception de la solution de bain usagée (13) représente une conduite de liquide pour le transfert à un module d'élimination séparé du dispositif de bain.

**13.** Dispositif de bain médical selon une des revendications précédentes, **caractérisé en ce que** le dispositif de bain présente des rouleaux (15) ou roues côté sol.

**14.** Dispositif de bain médical selon une des revendications précédentes, **caractérisé en ce que** la solution de bain contenant des substances actives (8) est une solution de bain contenant du monoxyde d'azote (NO).

**15.** Utilisation d'un dispositif de bain médical selon une des revendications 1 à 14 pour la fabrication d'une solution de bain contenant du NO, dans laquelle le procédé pour la fabrication de la solution de bain contenant du NO comprend les étapes suivantes :

(a) mise à disposition d'une solution de bain comprenant au moins un donneur de NO au pH instable ;
(b) réglage de la valeur de pH de la solution de bain sur une valeur de pH qui induit la décomposition de l'au moins un donneur de NO au pH instable en formant du NO ;
(c) maintien d'une valeur de pH induisant la formation de NO pour une durée qui permet la formation d'une quantité de NO pertinente du point de vue physiologique ;
(d) augmentation de la valeur de pH de la solution de bain d'au moins un niveau de valeur de pH.

**16.** Utilisation du dispositif de bain médical selon la revendication 15, **caractérisée en ce qu'**après l'étape (d) ou (e), la solution de bain est irradiée de lumière pour la décomposition photolytique du donneur de NO en formant du NO.

**17.** Dispositif de bain médical selon une des revendications 1 à 14 pour l'utilisation lors du traitement ou de la prévention de maladies, **caractérisé en ce que** l'au moins une extrémité corporelle du patient est exposée au NO libérée du dispositif de bain, dans lequel le dispositif de douche peut être adapté à l'application respective.

**18.** Dispositif de bain médical selon la revendication 17, **caractérisé en ce que** la maladie est sélectionnée parmi le

groupe contenant des douleurs neuropathiques, varices, ischémies et maladies thrombopathiques, allergies, infections cutanées, inflammations cutanées, dermatite atopique notamment neurodermite, dermatomyosite et pemphigus vulgaire ; défauts de lésion comme l'ulcère diabéto-neuropathique chronique, l'ulcère de la jambe, les lésions de décubitus ; lésions primaires, lésions infectées secondaires, complications lors de greffes cutanées, dysfonctionnement érectile, hidradénite suppurée (acné inversa), verrues, érythème fessier, brûlure de rasoir, syndrome de Raynaud, syndrome de Buerger, maladie artérielle périphérique (PAD), maladie d'occlusion artérielle périphérique (pAVK), maladies inflammatoires et auto-immunes de la peau (psoriasis, dermatites, neurodermite), maladies fongiques de la peau, maladies bactériennes, mycosiques et parasitaires de la peau (par ex. leishmaniose), tinea cruris, tinea inguinalis, dystrophies musculaires, drépanocytose et alopécie, dans lequel le dispositif de douche peut être adapté à l'application respective.

**19.** Dispositif de bain médical selon la revendication 18, **caractérisé en ce qu'**il est utilisé pour le traitement de lésions chroniques des extrémités inférieures de personnes diabétiques, dans lequel le dispositif de douche peut être adapté à l'application respective.

**20.** Procédé cosmétique comprenant l'action du NO sur la peau d'un homme, **caractérisé en ce qu'**un dispositif de bain médical selon une des revendications 1 à 14 est utilisé pour la fabrication d'une solution de bain contenant du NO pour l'action du NO sur la peau d'un homme.

Fig. 1

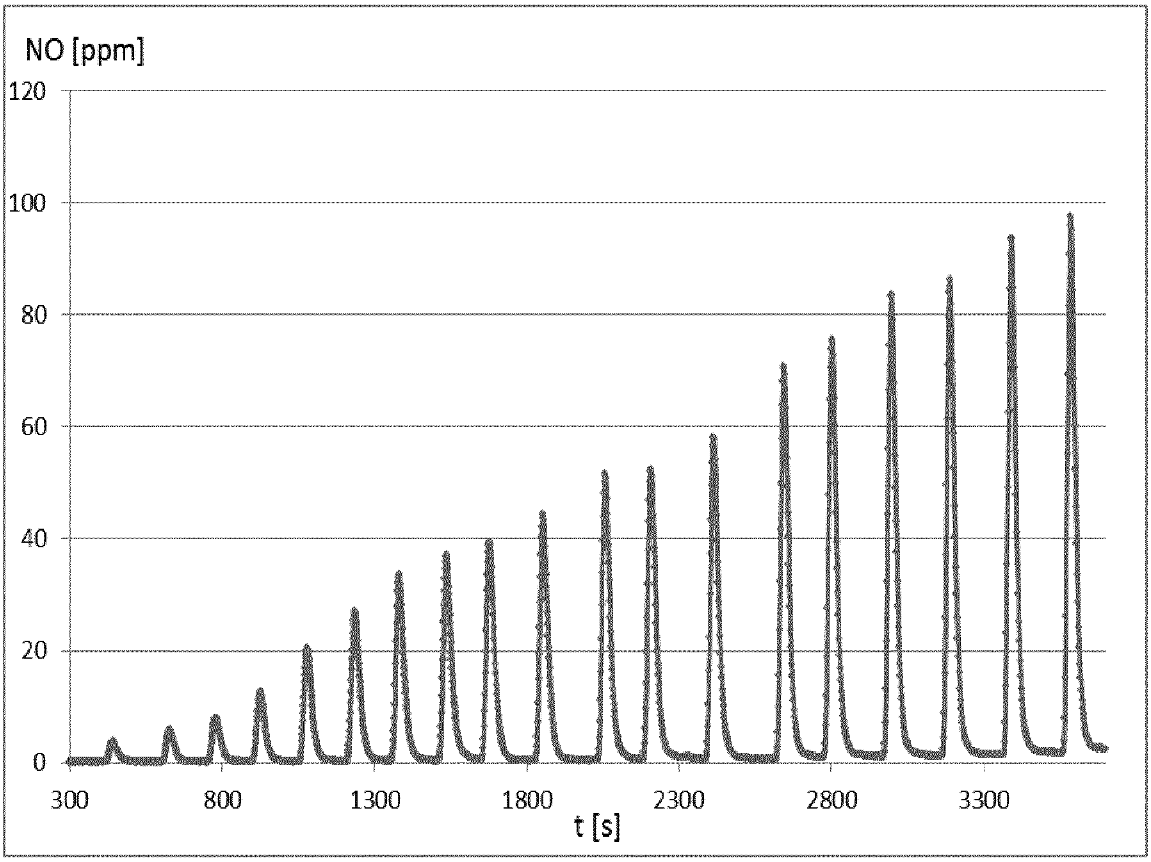

**Fig. 2**

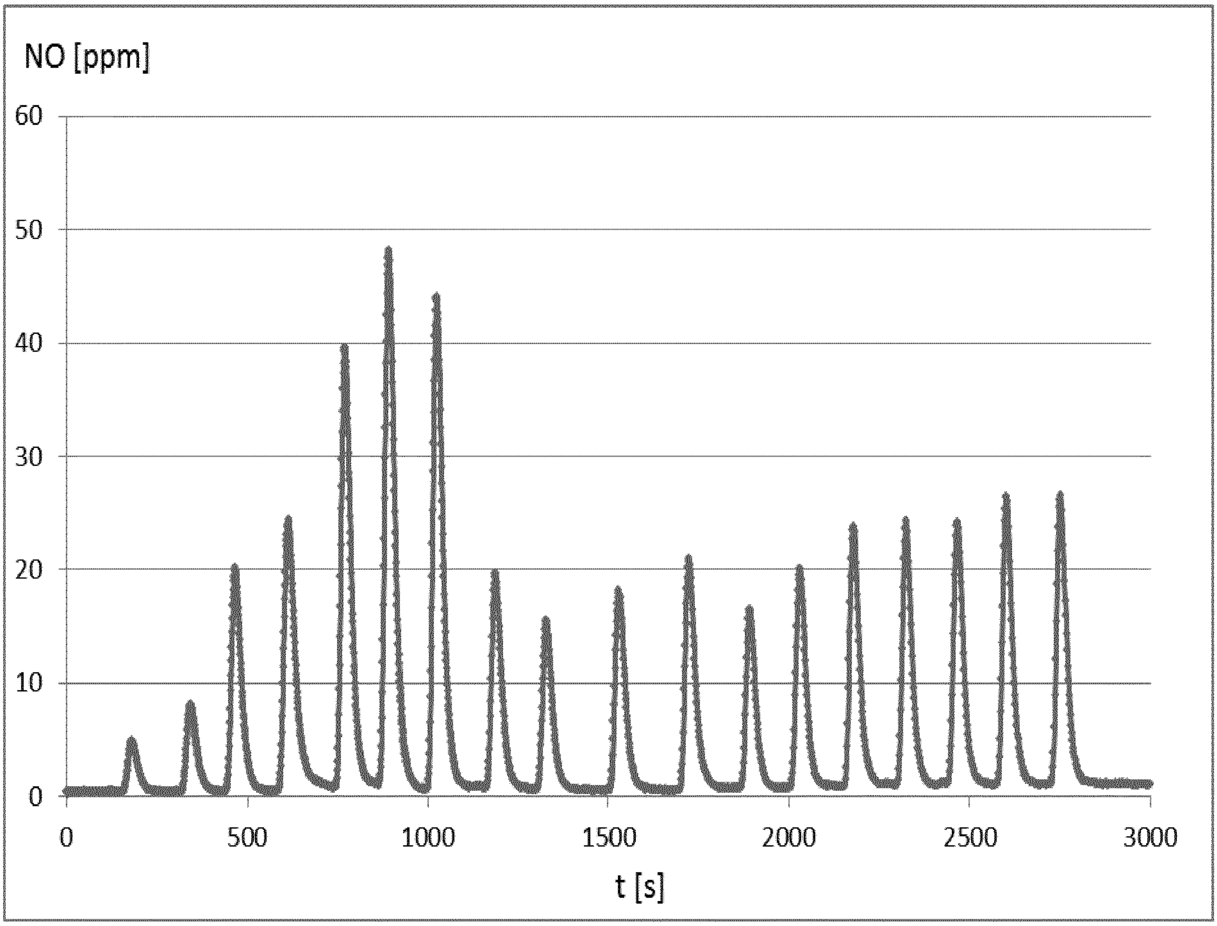

**Fig. 3**

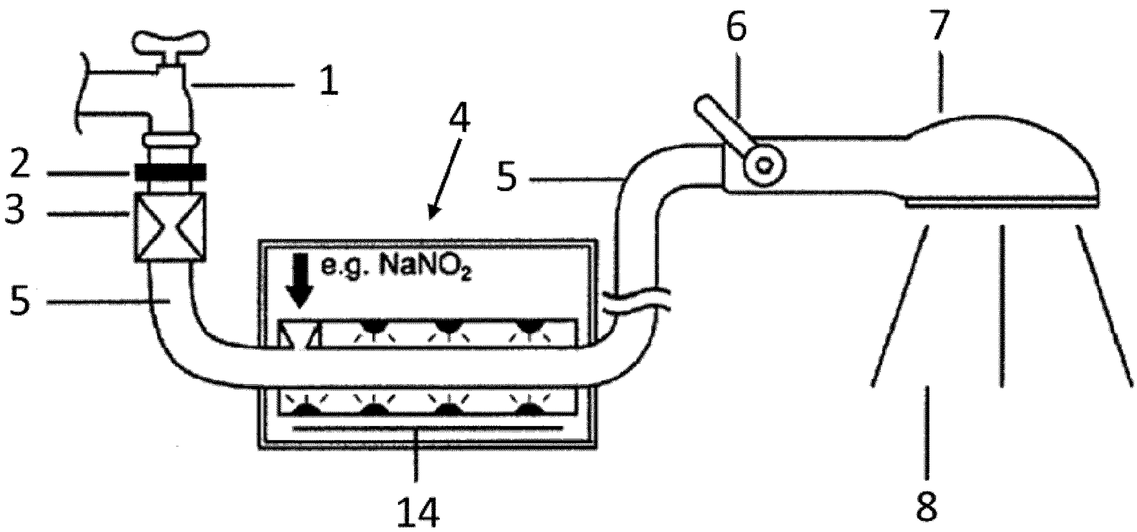

Fig. 4

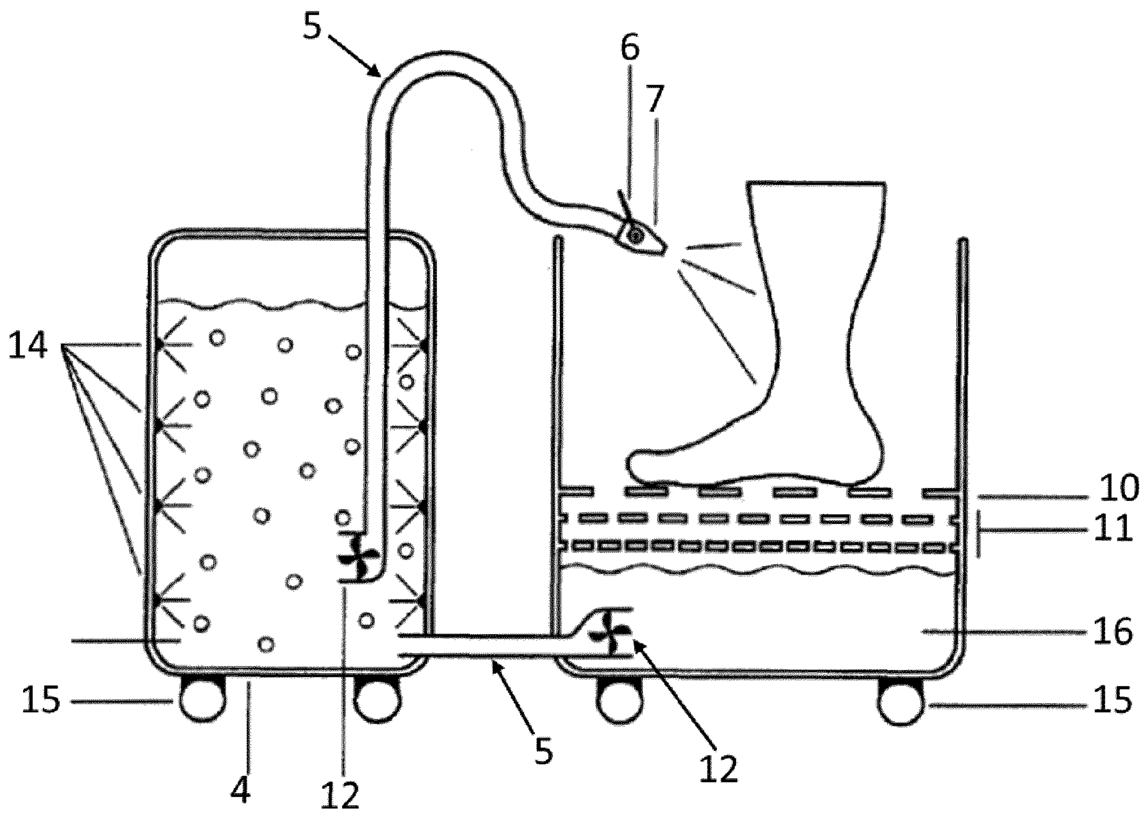

**Fig. 5**

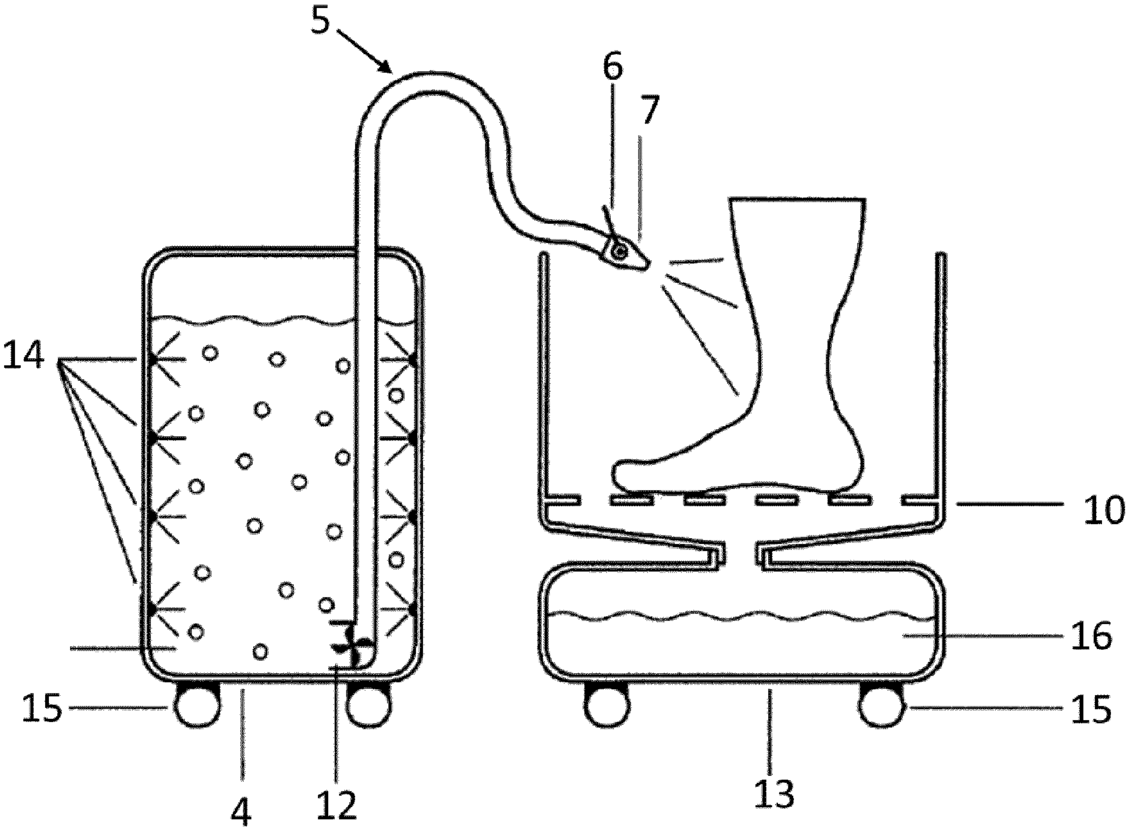

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2013040415 A1
- EP 1903003 A1
- WO 2013063354 A
- WO 9746853 A
- US 7105502 B
- US 7122529 B
- US 6673338 B
- US 20130224083 A1
- US 20030039697 A
- US 4151175 A
- US 3862333 A
- US 4694029 A
- EP 562897 A
- US 4399071 A
- WO 9846647 A
- US 5237059 A
- EP 571330 A
- EP 241423 A
- EP 139609 A
- EP 361907 A
- EP 199672 A
- EP 48615 A
- EP 12158 A
- EP 780729 A
- WO 20001097 A2
- GB 2348644 A
- WO 0026219 A
- WO 02101462 A1
- WO 2005097876 A1
- WO 2003050912 A
- EP 0898202 A1
- WO 9428075 A1
- WO 0192362 A1
- EP 0970085 A1
- WO 03033500 A1
- GB 2307473 A
- WO 9428075 A
- WO 9731033 A
- WO 199838195 A
- WO 200010964 A
- WO 199832756 A
- WO 199841524 A


### In der Beschreibung aufgeführte Nicht-Patentliteratur


- **KIRSCH et al.** *Biol. Chem,* 2002, vol. 383, 389-399
- Photoinitiators for Free Radical, Cationic & Anionic Photopolymerisation. **J.V. CRIVELLO ; K. DIETLIKER.** Chemistry & Technology of UV & EB Formulation for Coatings, Inks & Paints. J. Wiley and Sons/SITA Technology, 1998, vol. III, 464-466
- **M. SHIRAI ; M. TSUNOOKA.** *Prog. Polym. Sci.,* 1996, vol. 21, 1-45